(19) Europäisches Patentamt
European Patent Office
Office européen des brevets

(11) EP 1 717 219 A1

# (12) EUROPEAN PATENT APPLICATION

(43) Date of publication:
**02.11.2006 Bulletin 2006/44**

(21) Application number: **05009363.2**

(22) Date of filing: **28.04.2005**

(51) Int Cl.:
*C07C 217/22* (2006.01) *C07C 269/04* (2006.01)
*C07C 269/06* (2006.01) *C07K 7/06* (2006.01)
*C07K 7/52* (2006.01)

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(71) Applicant: **Schering AG**
**13342 Berlin (DE)**

(72) Inventors:
- **Srinivasan, Ananth**
  **10629 Berlin (DE)**
- **Luyt, Leonard G.**
  **London, ON N6C1L6 (CA)**

(74) Representative: **Krauss, Jan et al**
**Forrester & Boehmert,**
**Pettenkoferstrasse 20-22**
**80336 München (DE)**

## (54) Orthogonally protected bifunctional amino acid

(57) The present invention concerns novel orthogonally protected amino acids, there production and use for the synthesis of binding compounds usable in the diagnosis and treatment of proliferative diseases, in particular tumor diseases.

**Fig. 1**

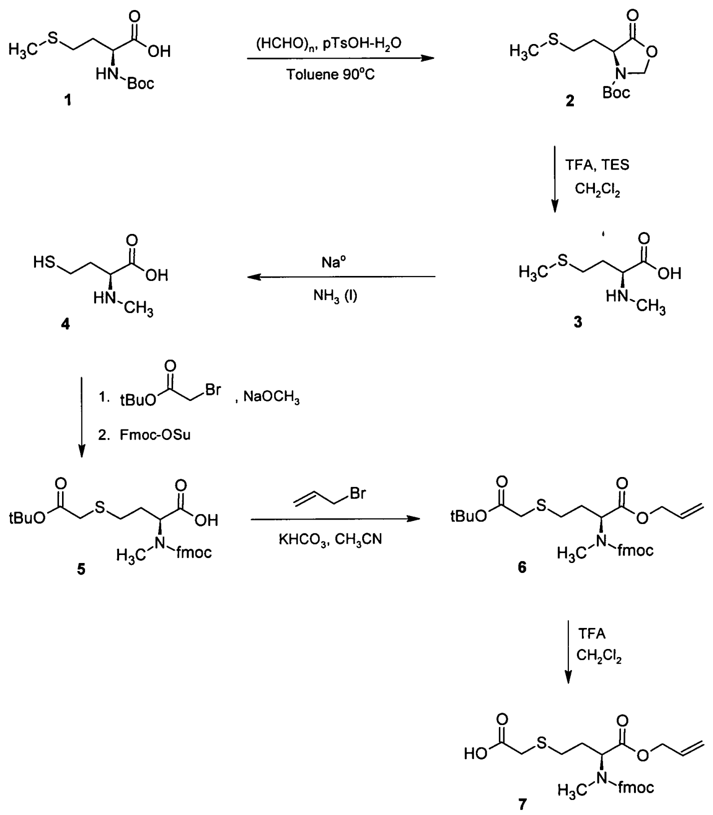




Printed by Jouve, 75001 PARIS (FR)

## Description

The present invention concerns novel orthogonally protected amino acids, their production and use for the synthesis of binding compounds usable for diagnosis and treatment of a proliferative diseases, in particular tumor diseases, infectious diseases, vascular diseases, rheumatoid diseases, inflammatory diseases, immune diseases, in particular autoimmune diseases and allergies..

## Background of the invention

Many human and animal diseases are characterized by an alteration of properties of diseased cells or cells that are in the vicinity of the diseased regions. The alterations include the loss of expression of proteins, the expression of mutated or truncated proteins as well as the untimely expression of proteins. An example of diseased cells, which show an alteration of the expression of proteins are tumor cells, which inappropriately express receptors for growth factors, e.g. epidermal growth factor receptor (EGFR) or vascular growth factor receptor (VEGFR), express mutated receptors, e.g. Her2, or cytoplasmic proteins including, e.g. p53 or pRb. One receptor commonly expressed by tumor cells, which is usually not expressed by healthy cells is a receptor bound by the peptide hormone somatostatin. An example of cells in the vicinity of diseased regions, which show an alteration of the expression of proteins are endothelial cells in tumor tissue, i.e. the tumor endothelium, which express certain proteins like, e.g. oncofoetal fibronectin or vascular endothelial growth factor (VEGF) normally not expressed by endothelial cells. Molecular structures that are preferentially or exclusively present in or in the vicinity of tumor cells have been described (for a review see, for example, Alessi P, et al. (2004) Biochim. Biophys. Acta. 1654:39-49 and Nanda A and St. Croix B (2004) Curr. Opin. Oncol. 16:44-49).

It is a well recognized fact that these alterations in particular the alterations of protein expression observed in diseased tissues can serve as a means for specifically recognizing and/or targeting substances to the diseased tissue or cells or to tissue or cells in the vicinity of the diseased tissue. In order to achieve effective binding to, for example, receptors exclusively or primarily expressed on tumor cells it is necessary that the binding component used to target the diseased tissue is capable of high affinity binding to the respective receptor. In many cases the altered or extemporary expressed surface structures, in particularly receptors, specifically recognize or are recognized by certain peptide or protein ligands. Theoretically, one could use these peptide or protein ligands to specifically target the cells or tissue. However, it is often not feasible to use the full length peptide or protein in an approach to target the diseased tissue due to, e.g. instability of the full length peptide or protein, the high costs associated with production and/or due to problems associated with formulation and administration of large peptides and proteins.

One approach to overcome the problems associated with the use of peptide and protein drugs has been the replacement of amino acids with so called peptidomimetics, which are amino acid analogues having a size and charge distribution similar to the encoded amino acid. Another approach has been the identification of small binding peptides. However, while produced more easily such small peptides can still have significant stability problems, which make them unsuitable for targeting purposes. The stabilization of small binding peptides has been achieved in the past through, e.g. N-terminal and/or C-terminal modification or cyclization. Cyclization can lead to peptides which on one hand maintain the three dimensional structure of the key interacting amino acids and on the other hand are more stable both inside and outside the body and, thus, more susceptible to pharmaceutical formulation and administration. U.S. 4,310,518, U.S. 4,486,415, EP 0 143 307 and EP 0 222 578 disclose, for example, cyclic hexapeptide somatostatin analogues which are cyclized through peptide linkages. U.S. 5,708,135 discloses somatostatin analogues which are cyclized through a disulfide bond between the N-terminal residues and the C-terminal residues. U.S. 5,770,687 discloses conformationally constrained backbone cyclized somatostatin analogues.

A receptor specific for the peptide hormone somatostatin is specifically and/or preferentially expressed on many tumors in particular on neuroendocrine tumors such as pituitary adenomas, pheochromocytomas, paragangliomas, some medulary thyroidcarcinomas and some small cell lung cancers. In addition cells of nervous system tumors such as astrocytomas and meningiomas display somatostatin receptors on their surfaces. Finally somatostatin receptor expression has also been found in human breast tumors, malignant lymphomas and renal cell carcinomas and some prostate tumors.

In addition to a cyclic peptide, which is one example of a binding component, which specifically recognizes a certain disease specific structure, i.e. a receptor, these binding compounds usually comprise one or more additional components which is (are) recruited to the cell or tissue via the specific binding component, e.g. the cyclized peptide. These components can include, for example, therapeutics and diagnostics, e.g. dyes, peptides, proteins, or metal chelating residues, which can bind a diagnostic or therapeutic isotope. In the past compounds comprising these two or more components, e.g. a peptide capable of specific surface structure recognition and metal chelating residues, were synthesized by classical linear solid phase peptide chemistry and then upon release of the linear peptide cyclized. This cyclic peptide was then conjugated to the second component. Thus the synthesis of the final binding compound requires the dissociation of the peptide from the solid phase and an in solution cyclization and coupling, which requires additional

manipulations of the reaction mixture.

The present inventors have now designed a new amino acid, which is a convenient starting compound in the synthesis of such binding compounds comprising at least two functionalities, e.g. a binding component (first component) and. a therapeutic or diagnostic component (second component), a reaction scheme employing these amino acids can be performed entirely on a solid phase, which makes the synthesis of therapeutic or diagnostic binding compounds more rapid and cost effective and provides additional advantages in the synthesis of certain cyclic peptides, which are difficult to synthesize with the conventional method.

Consequently, a first aspect of the present invention concerns an orthogonally protected bifunctional amino acid and salts thereof, which can form the basis for the synthesis of compounds comprising at least two components or functionalities as set out above.

The orthogonally protected bifunctional amino acid according to the present invention has a structure according to formula (I), (II) or (III):

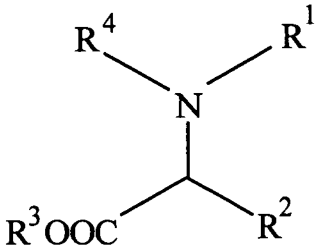
(I)

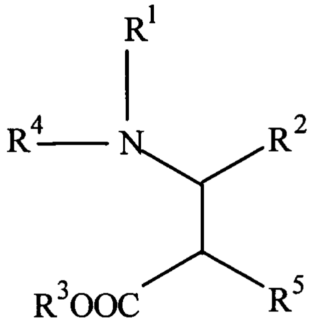
(II)

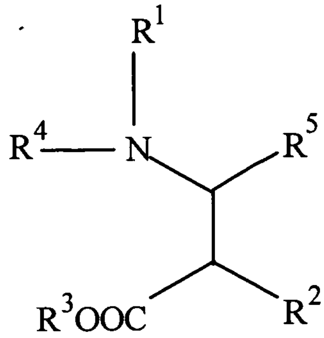
(III)

wherein,

$R^1$ and $R^2$ are independently of each other hydrogen, branched or linear $C_1$-$C_6$ alkyl, e.g. methyl, ethyl, propyl, isopropyl, n-butyl, isobutyl, pentyl or hexyl, branched or linear substituted $C_1$-$C_6$ alkyl, e.g. substituted e.g. methyl, ethyl, propyl, isopropyl, n-butyl, isobutyl, pentyl or hexyl, or $-CH_2-(CHY)_n-W-(CHY)_{n'}-X$, with the proviso that in formula (I) $R^1$ and $R^2$ are not hydrogen;

W is CHY, S, O, $N(CH_3)$, $N(C_2H_5)$ or $N(C_3H_7)$;

X is COOH, $NH_2$, COZ, NHZ or Z;

Y is for each CHY independently of each other hydrogen, methyl or halogen, preferably F, Cl or Br;

Z is an amino acid residue; a polypeptide; a protective group; which can be selectively removed in the presence of $R^3$ and $R^4$; a direct or indirect bond to a metal chelating residue, a dye, a therapeutic compound, or a surface; or a bond,

n is 0-6, e.g. 0, 1, 2, 3, 4, 5 or 6;

n' is 1-6, e.g. 1, 2, 3, 4, 5 or 6 or n' is 0-6, e.g. 0, 1, 2, 3, 4, 5 or 6, under the proviso that W is CHY;

$R^3$ is a protective group, which can be selectively removed in the presence of $R^4$;

$R^4$ is a protective group, which can be selectively removed in the presence of $R^3$; and

$R^5$ is hydrogen, branched or linear $C_1$-$C_6$ alkyl, e.g. methyl, ethyl, propyl, isopropyl, n-butyl, isobutyl, pentyl or hexyl, branched or linear substituted $C_1$-$C_6$ alkyl, e.g. substituted methyl, ethyl, propyl, isopropyl, n-butyl, isobutyl, pentyl or hexyl, or an amino acid side chain residue, preferably a side chain residue of asparagine, cystein, aspartic acid, glutamine, glutamic acid, phenylalanine, histidine, isoleucine, lysine, leucine, methionine, proline, arginine, serine, threonine, tryptophane, valine and tyrosine. The property of two protection groups to be capable of being selectively removed in the presence of the other and vice versa is known in the art as orthogonallity, i.e. the two protection groups are orthogonal to each other. The amino acids of the present invention carry orthogonal protection groups in this sense.

In a preferred embodiment the orthogonally protected bifunctional amino acid is an N-alkyl amino acid. If the N-residue in an amino acid according to formulas (I), (II) or (III) is alkyl substituted the residues $R^3$ and $R^4$ are more likely to be oriented in a cis-orientation and thus will more readily form a cyclic peptide compound. Thus, in a particular preferred embodiment $R^1$ is branched or linear $C_1$-$C_6$ alkyl, e.g. methyl, ethyl, propyl, isopropyl, N-butyl, iso-butyl, pentyl or hexyl, or branched or linear substituted $C_1$-$C_6$ alkyl, e.g. a substituted methyl, ethyl, propyl, isopropyl, n-butyl, isobutyl, pentyl or hexyl.

In a further preferred embodiment $R^2$ is branched or linear $C_1$-$C_6$ alkyl, e.g. methyl, ethyl, propyl, isopropyl, n-butyl, isobutyl, pentyl or hexyl, branched or linear substituted, e.g. substituted methyl, ethyl, propyl, isopropyl, n-butyl, isobutyl, pentyl or hexyl, or $-CH_2-(CHY)_n-W-(CHY)_{n'}-X$. In this context $R^5$ is preferably hydrogen or an amino acid side chain residue. An even more preferred orthogonally protected bifunctional amino acid according the present invention is an amino acid, wherein $R^1$ is branched or linear $C_1$-$C_6$ alkyl, e.g. methyl, ethyl, propyl, isopropyl, n-butyl, isobutyl, pentyl or hexyl, or branched or linear substituted $C_1$-$C_6$ alkyl, e.g. substituted methyl, ethyl, propyl, isopropyl, n-butyl, isobutyl, pentyl or hexyl, and $R^2$ is $-CH_2-(CHY)_n-W-(CHY)_n-X$, Again in this context it is preferred that $R^5$ is a hydrogen or an amino acid side chain residue.

In a preferred embodiment of the orthogonally protected bifunctional amino acid according to the present invention W is S, O or $N(CH_3)$. If W has the preferred meaning as indicated in the preceding sentence it is further preferred that $R^1$ has its preferred meaning, i.e. $R^1$ is branched or linear $C_1$-$C_6$ alkyl, e.g. methyl, ethyl, propyl, isopropyl, n-butyl, isobutyl, pentyl or hexyl, or branched or linear substituted $C_1$-$C_6$ alkyl, e.g. substituted methyl, ethyl, propyl, isopropyl, n-butyl, isobutyl, pentyl or hexyl. In an even more preferred embodiment W and $R^1$ have the preferred meaning outlined in this para. and $R^5$ is hydrogen or an amino acid side chain residue.

The group Z within the orthogonally protected amino acid of the present invention represents a bond, or a second component or a part thereof, which will be present in the binding compound, which is the product of the synthesis starting with the orthogonally protected amino acid of the present invention, e.g. the second component is a polypeptide, a dye, a therapeutic or a metal chelating residue. Preferably this further component is already present in the orthogonally protected amino acid, when the synthesis of the first component is started. The first component will be attached to the amino and/or carboxy residue(s) protected by $R^3$ and $R^4$, respectively. Thus in a preferred embodiment Z can be any naturally or non-naturally occurring amino acid it is, however, even more preferred when Z is selected from the group consisting of alanine-A, asparagine-A, cystine-A, asparagine-A, aspartic acid-A, glutamine-A, glutamic acid-A, phenylalanine-A, glycine-A, histidine-A, isoleucine-A, lysine-A, leucine-A, methionine-A, proline-A, arginine-A, serine-A, threonine-A, tryptophane-A, valine-A, tyrosine-A, tert-butyl glycine-A, N-methyl phenylalanine-A, lysine(GlyMeDOTA)-A Hcy-A, Hhc-A, Pen-A, Aib-A, Nal-A, Aca-A, Ain-A, Hly-A, Achxa-A, Amf-A, Aec-A, Apc-A, Aes-A, Aps-A, Abu-A, Nva-A, FD-A, WD-A, YD-A, Cpa-A, Thp-A, D-Nal-A, Dpg-A, Dab-A, Nle-A, $(N-CH_3)$Cys-A, Om-A, $(N-CH_3)$Hcy-A, $(N-CH_3)$Tyr-A, $(N-CH_3)$Tty-A, $(N-CH_3)$Tyr-A($CH_2$ $CH_2$ SH), Thr(OH)-A, Ser(ol)-A, Asp(ol)-A, Glu(ol)-A, Gln(ol)-A, Asn(ol)-A, Phe(4-F)-A, Phe(4-$NH_2$)-A, ε-Lys-A, δ-Orn-A, γ-Dab-A, β-Dap-A. In order to prevent the modification of amino acid side chains during subsequent couplings/reactions involving the $R^3$ protected carboxyl residue and the $R^4$ protected amino residue the amino acids can optionally comprise (a) protected side chain residue(s). This residue will preferably not be cleaved under conditions that cleave $R^3$ and/or $R^4$.

Furthermore, within the above indicated amino acids "A" is the amino or carboxyl group of the amino acid, a protected amino or carboxyl group or a direct or indirect bond to a surface.

The term "direct bond" in this context and as used throughout the specification means a covalent or non-covalent bond to a further residue, i.e. a direct bond to a surface is a covalent bond to a residue attached to the surface. The term "indirect bond" as used herein means that one or more additional chemical residues, which are attached via covalent or non-covalent bonds to the amino acid are located between the amino acid and a surface. These one or more additional chemical residues can also be termed "spacer". A spacer can, e.g. provide a spatial separation between the surface and the orthogonally protected amino acid of the present invention to prevent or reduce, e.g. phenomenons associated with the interface of the solid and the liquid medium.

The term "surface" refers to the interphase of a gaseous or liquid medium with a solid or semi-solid medium. The solid medium preferably includes but is not limited to glass, metal, artificial or natural polymers, in particular polyvinyl chloride, polyethylene, polypropylene, poly urethanes, polystyrols, polyamids, polyesters, polysaccharides, polytetrafluorethylene and the like. The surface can have any form but is preferably a smooth or porous surface which is shaped in any suitable form including, e.g. beads, cylinders and the like.

In a further preferred embodiment Z can be a polypeptide. The term "polypeptide" is used to refer to polyamino acids with two or more amino acid residues and, thus, includes peptides, a term which is often used to refer to polyamino acids with 2 to 100 amino acids, and proteins, a term which is often used to refer to polyamino acids with more than 100 amino acids. A polypeptide component can comprise naturally and non-naturally occurring amino acids in particular alanine, asparagine, cystine, asparagine, aspartic acid, glutamine, glutamic acid, phenylalanine, glycine, histidine, isoleucine, lysine, leucine, methionine, proline, arginine, serine, threonine, tryptophane, valine, tyrosine, tert-butyl glycine, N-methyl phenylalanine, lysine(GlyMeDOTA) Hcy, Hhc, Pen, Aib, Nal, Aca, Ain, Hly, Achxa, Amf, Aec, Apc, Aes, Aps, Abu, Nva, FD, WD, YD, Cpa, Thp, D-Nal, Dpg, Dab, Nle, $(N-CH_3)$Cys, Orn, $(N-CH_3)$Hcy, $(N-CH_3)$Tyr, $(N-CH_3)$Tty, $(N-CH_3)$Tyr($CH_2$ $CH_2$ SH), Thr(OH), Ser(ol), Asp(ol), Glu(ol), Gln(ol), Asn(ol), Phe(4-F), Phe(4-$NH_2$), ε-Lys, δ-Orn, γ-Dab, β-Dap. Again it is preferred that the terminal amino acid is linked via its amino and carboxy terminus, respectively, directly or indirectly, e.g. with an intermittent spacer, to a surface.

In a preferred embodiment the polypeptide is selected from the group consisting of a receptor ligand, an antibody, a single chain antibody or a binding fragment of an antibody or single chain antibody. The term antibody comprises fully human, humanized, chimeric and xenogenic antibodies. The binding fragments of an antibody, are

preferably antibody binding domain fragments, e.g. Fv, Fab, Fab', F(ab')$_2$, Fabc, Facb. The term "single chain" antibody comprises, e.g. single chain Fvs (scFvs) and diabody.

In a preferred embodiment the second component of the substance resulting from the synthesis employing the orthogonally protected amino acid of the present invention is capable of chelating metals, in particular metal ions. A large variety of such metal chelating moieties are known in the art and are described in, for example, US 5,654,272, US 5,681,541, US 5,788,960, US 5,811,394, US 5,720,934, US 5,776,428, US 5,780,007, US 5,922,303, US 6,093,383, US 6,086,849, US 5,965,107, US 5,300,278, US 5,350,837, US 5,589,576, US 5,679,778 and US 5,879,659. The respectively described metal chelating residues are specifically referenced herewith and can all equally be used as metal chelating residues in the context of the amino acid of the present invention. It should also be pointed out that some metal chelating residues can also be considered polypeptides as defined above and, thus, the term chelating residues overlaps with the term "polypeptides" in as far as the polypeptide has the capability to chelat metal, in particular metal ions.

In a preferred orthogonally protected bifunctional amino acid the metal chelating residue is selected from the group consisting of:

a) -C(PGP)$^s$-(aa)-C(PGP)$^s$, wherein (PGP)$^s$ is hydrogen or a thiol protecting group and (aa) is any [alpha]- or [beta]-amino acid not comprising a thiol group;

b) a substance according to formula (IV) or (V)

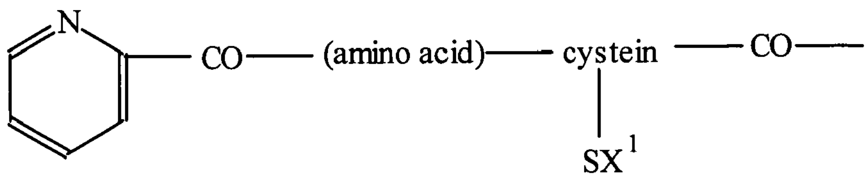

(IV)

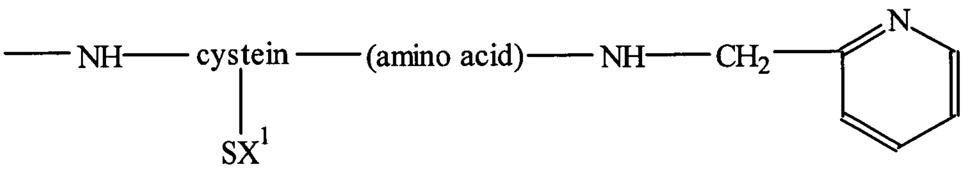

(V),

wherein X$^1$=H or a protecting group;
(amino acid)=any amino acid;

c) a substance according to formula (VI)

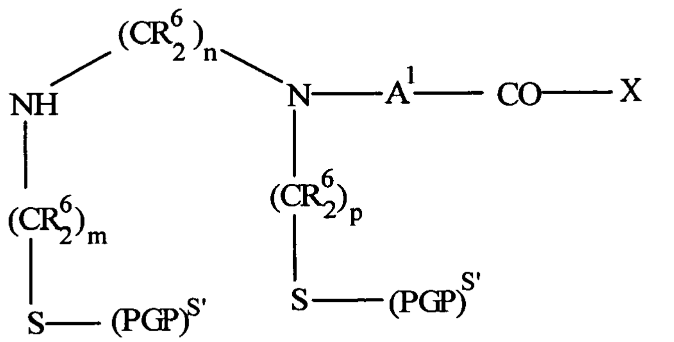

(VI),

wherein each $R^6$ is independently of each other H, $CH_3$ or $C_2H_5$, each $(PGP)^{s'}$ is independently a thiol protecting group or H; m, n and p are independently 2 or 3; $A^1$ is linear $C_1$-$C_8$ alkyl, e.g. methyl, ethyl, propyl, isopropyl, n-butyl, isobutyl, pentyl, hexyl, heptyl or octyl, substituted linear $C_1$-$C_8$ alkyl, e.g. substituted methyl, ethyl, propyl, isopropyl, n-butyl, isobutyl, pentyl, hexyl, heptyl or octyl, cyclic $C_3$-$C_8$ alkyl, e.g. cyclic propyl, butyl, pentyl, hexyl, heptyl or octyl, substituted cyclic $C_3$-$C_8$ alkyl, e.g. substituted cyclic propyl, butyl, pentyl, hexyl, heptyl or octyl, aryl, substituted aryl, or a combination thereof; and

d) a substance according to formula (VII)

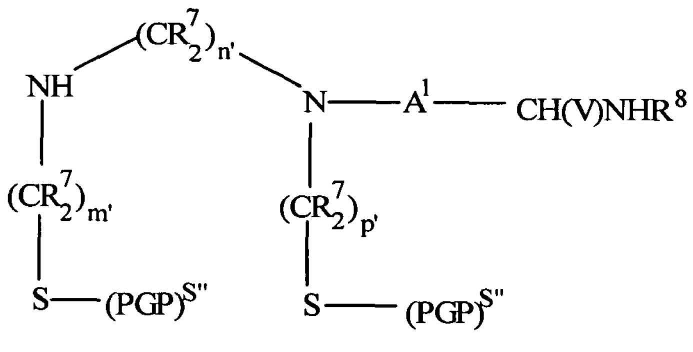

(VII),

wherein each $R^7$ is independently of each other H, $CH_3$ or $C_2H_5$; each (PGP)S" is independently a thiol protecting group or H; m', n' and p' are independently 2 or 3; $A^2$ is linear $C_1$-$C_8$ alkyl, e.g. methyl, ethyl, propyl, isopropyl, n-butyl, isobutyl, pentyl, hexyl, heptyl or octyl, substituted linear $C_1$-$C_8$ alkyl, e.g. substituted methyl, ethyl, propyl, isopropyl, n-butyl, isobutyl, pentyl, hexyl, heptyl or octyl, cyclic $C_3$-$C_8$ alkyl, e.g. cyclic propyl, butyl, pentyl, hexyl, heptyl or octyl, substituted cyclic $C_3$-$C_8$ alkyl, e.g. substituted cyclic propyl, butyl, pentyl, hexyl, heptyl or octyl, aryl, substituted aryl, or a combination thereof; V is H or CO-X; $R^8$ is H or a direct or indirect bond, preferably covalent bond, to X; under the proviso that when $R^8$ is H than V is preferably CO-X.

e) diethylenetriaminepentaacetic acid (DTPA);

f) a derivative of DTPA having a formula (VIII)

$$(HOOCCH_2)_2N(CR^9_2)(CR^9_2)N(CH_2COOH)(CR^9_2)(CR^9_2)N(CH_2COOH)_2$$

(VIII),

wherein each $R^9$ is independently of each other H, $C_1$ to $C_4$ alkyl, e.g. methyl, ethyl, n-propyl, iso-propyl, n-butyl, iso-butyl, or aryl and one $R^9$ is a direct or indirect bond, preferably covalent bond, to X;

g) ethylenediaminetetraacetic acid (EDTA);

h) a derivative of EDTA having a formula (IX)

$$(HOOCCH_2)_2N(CR^{10}_2)(CR^{10}_2)N(CH_2COOH)_2$$

(IX),

wherein each $R^{10}$ is independently H, $C_1$ to $C_4$ alkyl, e.g. methyl, ethyl, n-propyl, iso-propyl, n-butyl, iso-butyl, or aryl and at least one $R^{10}$ is a direct or indirect bond, preferably covalent bond, to X;

i) 1,4,7,10-tetraazacyclododecanetetraacetic acid and derivatives thereof;

j) a substance according to formula (X)

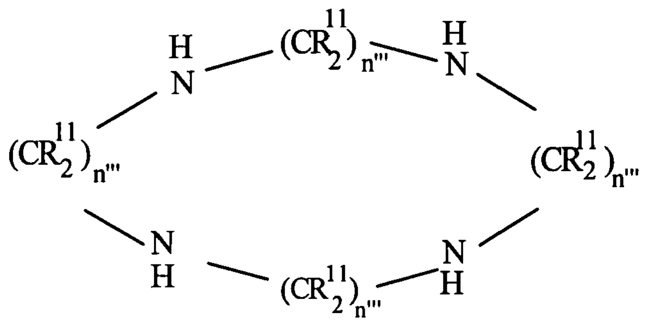

(X),

wherein n"' is an integer that is 2 or 3 and where each $R^{11}$ is independently H, $C_1$ to $C_4$ alkyl, or aryl and one $R^{11}$ is a direct or indirect bond, preferably covalent bond,to X;

k) a substance according to formula (XI) comprising a single thiol

$$A^3\text{-}CZ^3(B^3)\text{-}\{C(R^{12}R^{13}\}_{n''}\text{-}X^3 \qquad (XI),$$

wherein $A^3$ is H, HOOC-, $H_2NOC$-, -NHOC-, -OOC-, $R^{16}{}_2NOC$-, X-NHOC-, X-OOC-, or $R^{15}$; $B^3$ is H, SH, -$NHR^{14}$, -$N(R^{14})$-, X-$NR^{14}$- or $R^{15}$, $Z^3$ is H or $R^{15}$; $X^3$ is SH,-$NHR^{14}$, -$N(R^{14})$-, X-$NR^{14}$- or $R^{15}$; $R^{12}$, $R^{13}$, $R^{14}$ and $R^{15}$ are independently H, straight chain $C_1$-$C_8$ alkyl, e.g. methyl, ethyl, propyl, isopropyl, n-butyl, isobutyl, pentyl, hexyl, heptyl or octyl, branched chain $C_1$-$C_8$ alkyl, or cyclic $C_3$-$C_8$ alkyl, e.g. propyl, butyl, pentyl, hexyl, heptyl or octyl; n" is 0, 1 or 2; $R^{16}$ is $C_1$-$C_4$ alkyl, an amino acid, or a peptide comprising 2 to about 10 amino acids; and: (1) where $B^3$ is -$NHR^{14}$, X-$NR^{14}$- or -$N(R^{14})$-, $X^3$ is SH and n" is 1 or 2; (2) where $X^3$ is -$NHR^{14}$, X-$NR^{14}$-, or - $N(R^{14})$-, $B^3$ is SH and n" is 1 or 2; (3) where $B^3$ is H or $R^{15}$, $A^3$ is HOOC-, $H_2NOC$-, X-NHOC-, X-OOC-, -NHOC-, or -OOC-, $X^3$ and n" is 0 or 1; (4) where $A^3$ is H or $R^{15}$, in cases where $B^3$ is SH, $X^3$ is -$NHR^{14}$, X-$NR^{14}$-, or -$N(R^{14})$- and where $X^3$ is SH, $B^3$ is -$NHR^{14}$, X-$NR^{14}$- or -$N(R^{14})$ and n" is 1 or 2; (5) where $X^3$ is H or $R^{15}$, $A^3$ is HOOC-, $H_2NOC$-, -NHOC-, -OOC-, X-NHOC- or X-OOC- and $B^3$ is SH; (6) where $Z^3$ is methyl, $X^3$ is methyl, $A^3$ is HOOC-, $H_2NOC$-, -NHOC-, -OOC-, X-NHOC- or X-OOC- and $B^3$ is SH and n is 0; and (7) where $B^3$ is SH, $X^3$ is not SH and where $X^3$ is SH, $B^3$ is not SH, and

l) a substance according to formula (XII)

-βDap-Xaa-Cys-Zaa-A (XII),

wherein
Xaa is an L-α-amino acid;
Zaa is an α-amino acid, an α-amino acid amide, an aminoethylether, a β-aminol, or a peptide containing from two to ten α-amino acids, said peptide having a carboxyl terminal α-amino acid, α-amino acid amide, aminoethylether, or β-aminol, and A is the amino or carboxyl group of the amino acid, a protected amino or carboxyl group or a direct or indirect bond to a surface.

The chelating moieties mentioned above and in particular the preferred chelating moieties can optionally comprise one or more protected side chain residues. The side chains are protected to assure that during coupling reactions taking place at the carboxy residue protected by $R^3$ and at the amino residue protected by $R^4$ that the chelating moieties are not altered.

In a particular preferred embodiment of the orthogonally protected bifunctional amino acid the metal chelating residue is selected from the group consisting of:

a) -βDap-Phe-Cys-Thr-Ser-A;
b) -βDap-Tyr-Cys-Thr(ol)-A;
c) -βDap-Phe(4-F)-Cys-Thr(ol)-A;
d) -βDap-Phe(4-$NH_2$)-Cys-Thr-Ser-A;
e) -βDap-Dab-Cys-Thr-A;
f) -βDap-Phe(4-NH2)-Cys-Thr-A;
g) -βDap-Phe(4-NH2)-Cys-Thr(ol)-A;
h) -βDap-His-Cys-Thr(ol)-A;
i) -βDap-Arg-Cys-Thr(ol)-A;
j) -βDap-Gly-Cys-Lys-$NH_2$-A;
k) -βDap-Ser-Cys-Thr(ol)-A;
l) -βDap-Dab-Cys-Thr(ol)-A;
m) -βDap-Gly-Cys-Thr(ol)-A;
n) -βDap-Dab-Cys-Ser(ol)-A;
o) -βDap-Ser-Cys-Thr-NH($CH_2CH_2O$)$_2$ $CH_2CH_2$NH-A;
p) -βDap-Orn-Cys-Thr(ol)-A
q) -βDap-Dap-Cys-Thr(ol)-A;
r) -βDap-Lys-Cys-Thr(ol)-A; and
s) -βDap-Lys-Cys-NH-A;

Again the preferred chelating moieties can optionally comprise one or more protected side chain residues and A has the meaning as outlined above.

For diagnostic purposes it is also possible to use a dye as a second component. Such dye can, for, example, allow a better determination of the perimeters of a tumor during a surgical procedure or can be used in imaging techniques employing light of various wavelengths like, e.g., laser imaging. The term "dye" within the meaning of the present encompasses substances, which are capable of adsorbing light in the visible or invisible spectrum and which are preferably capable to emit light in the visible or invisible spectrum. Thus, preferred dyes are fluorescent dyes. The skilled person is aware of a large number of dyes, which are similarly suitable for imaging purposes, in particular in vivo imaging purposes, which include, for example, fluorescent dyes as described in WO 00/61194, WO 00/71162, WO 01/52746, WO 01/52743 and WO 01/62156.

For therapeutic purposes the amino acid of the present invention can also comprise a therapeutic agent. This agent can be any therapeutic agent and preferably includes, therapeutic agents which benefit from targeted delivery like, e.g. analgesics; antirheumatics; anthelminthics; antiallergics; antianemics; antiarrhythmics; antibiotics; angiogenesis inhibitors; antiinfectives; antidemenics (nootropics); antidiabetics; antidotes; antiemetics; antivertiginosics; antiepileptics; antihemorrhagics; antihypertonics; antihypotonics; anticoagulants; antimycotics; antitussive agents; antiviral agents; beta-receptor and calcium channel antagonists; broncholytic and antiasthmatic agent; chemokines; cytokines, in particular immune modulatory cytokines; mitogens; cytostatics; cytotoxic agents and prodrugs thereof; dermatics; hypnotics and sedatives; immunosuppressants; immunostimulants in particular activators of NF-κB, MAP kinases, STAT proteins and/or protein kinase B/Akt; peptide or protein drugs; in particular hormones and physiological or pharmacological inhibitors of mitogens, chemokines, or cytokines or their respective prodrugs. In a preferred embodiment the drug is selected from the group consisting of chemokines, cytokines, mitogens, cytostatics, cytotoxic agents and prodrugs

thereof, immunostimulants, peptide or protein drugs, in particular hormones and physiological or pharmacological inhibitors of mitogens, chemokines, or cytokines or their respective prodrugs.

Preferred cytostatic or cytotoxic drug are alkylating substances, anti-metabolites, antibiotics, epothilones, nuclear receptor agonists and antagonists, anti-androgens, anti-estrogens, platinum compounds, hormones and antihormones, interferons and inhibitors of cell cycle-dependent protein kinases (CDKs), inhibitors of cyclooxygenases and/or lipoxygenases, biogenic fatty acids and fatty acid derivatives, including prostanoids and leukotrienes, inhibitors of protein kinases, inhibitors of protein phosphatases, inhibitors of lipid kinases, platinum coordination complexes, ethyleneimenes, methylmelamines, trazines, vinca alkaloids, pyrimidine analogs, purine analogs, alkylsulfonates, folic acid analogs, anthracendiones, substituted urea, methylhydrazin derivatives. Cytostatic or cytotoxic drugs comprise without limitations acediasulfone, aclarubicine, ambazone, aminoglutethimide, L-asparaginase, azathioprine, bleomycin, busulfan, calcium folinate, carboplatin, carpecitabine, carmustine, celecoxib, chlorambucil, cis-platin, cladribine, cyclophosphamide, cytarabine, dacarbazine, dactinomycin dapsone, daunorubicin, dibrompropamidine, diethylstilbestrole, docetaxel, doxorubicin, enediynes, epirubicin, epothilone B, epothilone D, estramucin phosphate, estrogen, ethinylestradiole, etoposide, flavopiridol, floxuridine, fludarabine, fluorouracil, fluoxymesterone, flutamide fosfestrol, furazolidone, gemcitabine, gonadotropin releasing hormone analog, hexamethylmelamine, hydroxycarbamide, hydroxymethylnitrofurantoin, hydroxyprogesteronecaproat, hydroxyurea, idarubicin, idoxuridine, ifosfamide, interferon $\alpha$, irinotecan, leuprolide, lomustine, lurtotecan, mafenide sulfate olamide, mechlorethamine, medroxyprogesterone acetate, megastrolacetate, melphalan, mepacrine, mercaptopurine, methotrexate, metronidazole, mitomycin C, mitopodozide, mitotane, mitoxantrone, mithramycin, nalidixic acid, nifuratel, nifuroxazide, nifuralazine, nifurtimox, nimustine, ninorazole, nitrofurantoin, nitrogen mustards, oleomucin, oxolinic acid, pentamidine, pentostatin, phenazopyridine, phthalylsulfathiazole, pipobroman, prednimustine, prednisone, preussin, procarbazine, pyrimethamine, raltitrexed, rapamycin, rofecoxib, rosiglitazone, salazosulfapyridine, scriflavinium chloride, semustine streptozocine, sulfacarbamide, sulfacetamide, sulfachlopyridazine, sulfadiazine, sulfadicramide, sulfadimethoxine, sulfaethidole, sulfafurazole, sulfaguanidine, sulfaguanole, sulfamethizole, sulfamethoxazole, co-trimoxazole, sulfamethoxydiazine, sulfamethoxypyridazine, sulfamoxole, sulfanilamide, sulfaperin, sulfaphenazole, sulfathiazole, sulfisomidine, staurosporin, tamoxifen, taxol, teniposide, tertiposide, testolactone, testosteronpropionate, thioguanine, thiotepa, tinidazole, topotecan, triaziquone, treosulfan, trimethoprim, trofosfamide, UCN-O1, vinblastine, vincristine, vindesine, vinblastine, vinorelbine, and zorubicin, or their respective derivatives or analogs thereof.

As already pointed out above the orthogonally protected amino acids of the present invention can form the starting point for the synthesis of molecules with two or more components. The first component is added to the carboxy residue protected by $R^3$ and/or the amino residue protected by $R^4$. Thus, in order to add, e.g. a monomeric building block to either $R^3$ or $R^4$ the protective groups $R^3$ and/or $R^4$ have to be removed. If it is desired that the addition of this new monomeric building block is restricted to the carboxy and/or amino residue it is preferred that additional protection groups are not removed under conditions removing $R^3$ and/or $R^4$. Consequently, in a preferred embodiment, when Z is an amino acid residue, a polypeptide residue or a chelating residue, the amino acid residue, the polypeptide or the metal chelating residue carries one or more protection group(s), which (is) are stable under conditions that remove $R_3$ and/or $R_4$.

In a further preferred embodiment of the orthogonally protected bifunctional amino acid of the present invention n is 1-3, e.g. 1, 2 or 3 and n' is 1-3, e.g. 1, 2 or 3.

Since the orthogonally protected amino acids of the present invention can be starting compounds for the synthesis of binding compounds with two or more functionalities it is required to remove the protective groups $R^3$ and/or $R^4$ in order to allow the addition of monomeric building blocks to the carboxy and/or amino residue. In order to allow directed addition to either the carboxy or the amino residue it is preferred that both residues are protected by dif ferent protective groups, which differ in the conditions required for their removal and which, thus, allow the removal of $R^3$ or $R^4$ without removing the respective other protective group. The skilled artisan is aware of a large variety of protective groups, which can be employed in organic synthesis. Protective groups (also called protecting groups) are reviewed in, for example, Wuts, P.G.M. and Greene, T.W., Protective Groups in Organic Chemistry, 3rd Ed., 1999; Wily & Sons Inc. and in Kocienski, P.J., Protecting groups. 2nd Ed., 2000, Thieme Medical Publishing. Protective groups are organized in these reference books according to the functionalities that are protected as well as according to the conditions which remove the respective protective groups selectively. Protective groups suitable for orthogonal protection of amino acids for peptide synthesis are also described in Albericio F. Peptide Science, Volume 55, Issue 2 , Pages 123 - 139, 3 Nov 2000, John Wiley & Sons, Inc.

In a preferred embodiment of the orthogonally protected bifunctional amino acid of the present invention, $R^3$ and $R^4$ are each selected from a different group of protective groups selected from the group of protective groups removable by a nucleophile, by acidic conditions, preferably under which the peptide is still bound to the resin, by hydrogenolysis, by mild base or by photolytic conditions.

Particularly preferred protective groups, which can be used in the orthogonally protected bifunctional amino acid of the present invention are

(i) a protective group removed at acidic conditions, preferably at a pH between 4 and 6, which is selected from the group consisting of Boc or Trityl protecting groups;

(ii) a protective group removed by a nucleophile, which is selected from the group consisting of Fmoc or Dde protecting groups;

(iii) a protective group removed by hydrogenolysis consisting of the allyl type, the tert-butyl type, the benzyl type or Dmab (4,4-dimethyl-2,6-dicyclohexylidene)-3-methylbutyl]-amino} benzyl ester;

(iv) a protective group removed by radiation, which is selected from the group consisting of nitroveratryloxy carbonyl, nitrobenzyloxy carbonyl, dimethyl dimethoxybenzyloxy carbonyl, 5-bromo-7-nitroindolinyl, o-hydroxy-α-methyl cinnamoyl, and 2-oxymethylene anthraquinone.

Particular combinations of protective groups for $R^3$ and $R^4$ are preferred. It is preferred that $R^3$ protecting the carboxy group is removable by hydrogenolysis; mild base or photolytic conditions and $R^4$ protecting the amino group is removable by a nucleophile or acidic conditions, preferably under conditions which allow the peptide to still be bound to the resin. Examples of such preferred combinations include protective groups removed by hydrogenolysis and by a nucleophile. In a particular preferred embodiment $R^3$ is removed by hydrogenolysis and $R^4$ is removed by a nucleophile.

Out of these combinations it is even more preferred that in the orthogonally protected bifunctional amino acid $R^3$ is selected from the group of protective groups consisting of a protective group of the allyl type, the tert-butyl type and the benzyl type and $R^4$ is selected from the group of protective groups consisting of Fmoc, Boc and Dde.

Although the orthogonally protected bifunctional amino acid of the present invention can exhibit any stereoisomery it is preferred that $R^2$ has an L configuration.

A particular preferred species of an orthogonally protected bifunctional amino acid of the present invention has the formula (XIII):

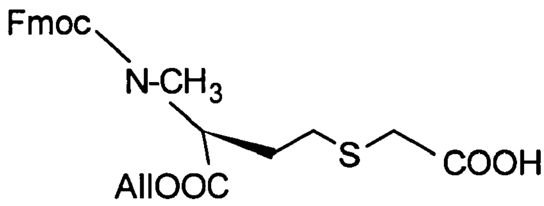

(XIII)

Methods for making the orthogonally protected bifunctional amino acids of the present invention are known to the skilled person and/or would be apparent to someone of skill based on the teaching contained herein. In particular, if $R^1$ or $R^2$ have the meaning $-CH_2-(CHY)_n-W-(CHY)_{n'}-X$, and Y has the meaning COZ, NHZ or Z and wherein Z is an amino acid, a polypeptide, a direct or indirect bond to a metal chelating residue, a dye, a therapeutic compound or a surface the second component, e.g. the polypeptide, the metal chelating residue, the dye, or the therapeutic compound can be synthesized independent from the first component and might only be linked to the amino acid residue after completion of the first component. However, even if the second component is only added after synthesis of the first component the amino acid of the present invention allows to synthesize the first component and, if desired cyclize the first component, e.g. the somatostatin receptor binding peptide, without detachment from the surface used for synthesis.

Methods for synthesizing the various preferred second components are well known in the art. Polypeptides, for example, are routinely synthesized on solid phase matrices. Similarly methods for making metal chelating residues are comprised in the previously cited patent literature. In particular US 5,443,815; US 5,807,537; US 5,814,297; US 5,866,097; US 5,997,844; US 6,074,627; WO 95/31221 and WO 95/33497 disclose the synthesis of preferred embodiments of metal chelators. The linkage of polypeptides to the amino acid of the present invention can be accomplished via peptide bonds, while metal chelators, dyes, therapeutic compounds or surfaces may be linked via carbon, nitrogen, sulphur or oxygen residues.

In a preferred embodiment the linkage of $(CHY)_{n'}-X$ to the amino acid is accomplished via the alkylation of (amino acid)-$CH_2-(CHY)_n$-W-(lower alkyl) with moieties containing reactive electrophiles such as alkyl halides, i.e. CHHa1-$(CHY)_{n'-1}$-X, wherein Hal has the meaning F, Cl, Br or I, preferably Cl or Br. An appropriately protected amino acid may also be linked to a metal chelator, a dye, a therapeutic compound or a surface through a side chain carbon by forming a Wittig or Emmons-Horner reagent on the carbon and reacting this with an aldehyde functionality on the metal chelator, dye, therapeutic compound or surface.

During attempts to synthesize the orthogonally protected bifunctional amino acids of the present invention it has been found by the present inventors that preferred compounds wherein $R^2$ has the meaning

$-CH_2-(CHY)_n-W-(CHY)_{n'}-X$ and wherein W is O or S can be synthesized efficiently and with high yields using an amino acid with either a ether or thioether group which is reacted with a halogenated alkane linked directly or indirectly, e.g. via $(CHY)_{n'}-X$, to Z.

This is a preferred method for introducing, Z, e.g. a polypeptide, a dye, metal chelator, therapeutic compound into the amino acid of the present invention. Thus, in a further aspect the present invention is directed at a method for producing the orthogonally protected bifunctional amino acid which comprises the step of reacting a compound of formula (XIV) to formula (XVI):

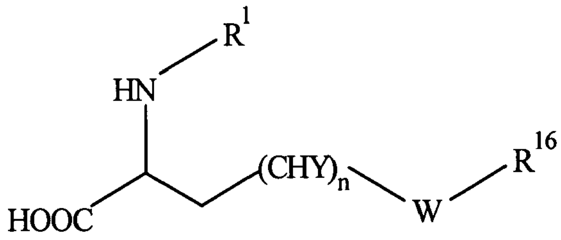

(XIV)

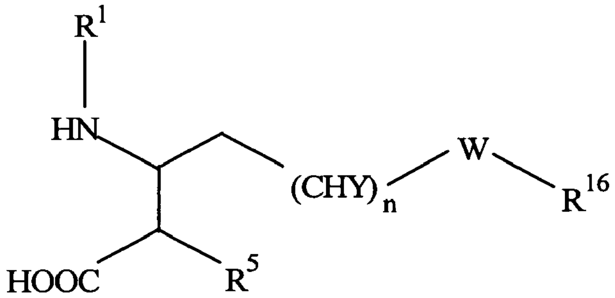

(XV)

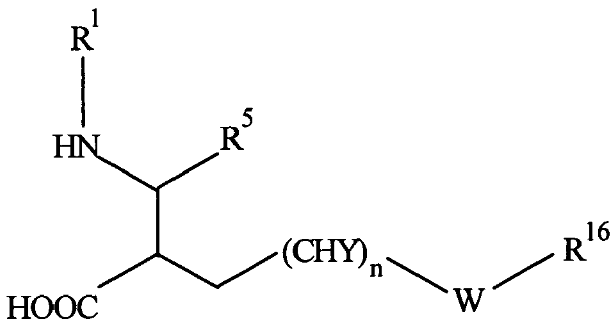

(XVI)

with

$$Hal-(CHY)_{n'}-X,$$

wherein $R^1$, $R^5$, X, Y, n and n' have the meaning as indicated above and in particular the indicated preferred meanings; W is O or S, $R^{16}$ is a leaving group, preferably $C_1$ to $C_6$ alkyl, e.g. methyl, ethyl, propyl, isopropyl, n-butyl, isobutyl, pentyl or hexyl, and Hal is F, Cl, Br, or I, preferably Cl or Br.

The amino acid of the present invention is a convenient compound to initiate the synthesis of binding compounds having two or more components. In many embodiments the amino acid of the present invention comprising a second

component will be synthesized attached to a solid surface and then additional steps to synthesise the first component can be carried out immediately without any detachment of the amino acid. Thus a further aspect of the present invention is a method for producing a binding compound comprising the step of

(i) selectively removing $R^3$ or $R^4$ from a orthogonally protected bifunctional amino acid and salts thereof having the formula (I), (II) or (III):

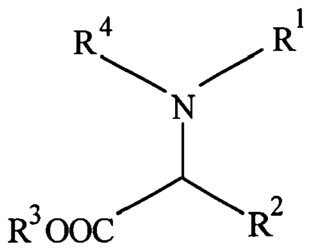

(I)

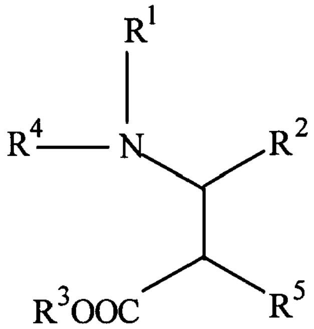

(II)

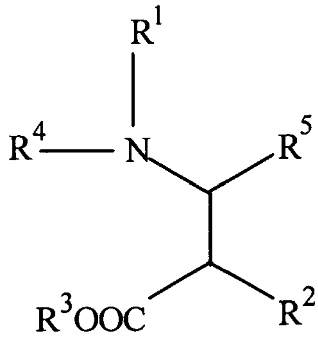

(III)

wherein,
$R^1$ and $R^2$ are independently of each other hydrogen, branched or linear $C_1$-$C_6$ alkyl, e.g. methyl, ethyl, propyl, isopropyl, n-butyl, isobutyl, pentyl or hexyl, branched or linear substituted $C_1$-$C_6$ alkyl, e.g. substituted methyl, ethyl, propyl, isopropyl, n-butyl, isobutyl, pentyl or hexyl, or $-CH_2-(CHY)_n-W-(CHY)_n-X$;
W is CHY, S, O, $N(CH_3)$, $N(C_2H_5)$ or $N(C_3H_7)$;
X is COOH, $NH_2$, COZ, NHZ or Z;
Y is for each CHY independently of each other hydrogen, methyl or halogen, preferably F, Cl or Br;
Z is an amino acid residue; a polypeptide; a protective group, which can be selectively removed in the presence of $R^3$ and $R^4$; a direct or indirect link to a metal chelating residue, a dye, a therapeutic compound or a surface; or a bond;
n is 0-6, e.g. 0, 1, 2, 3, 4, 5 or 6;
n' is 1-6, e.g. 1, 2, 3, 4, 5 or 6 or n' is 0-6, e.g. 0, 1, 2, 3, 4, 5 or 6, under the proviso that W is CHY;
$R^3$ is a protective group, which can be selectively removed in the presence of $R^4$;
$R^4$ is a protective group, which can be selectively removed in the presence of $R^3$; and
$R^5$ is hydrogen, branched or linear $C_1$-$C_6$ alkyl, e.g. methyl, ethyl, propyl, isopropyl, n-butyl, isobutyl, pentyl or hexyl, branched or linear substituted $C_1$-$C_6$ alkyl, e.g. substituted methyl, ethyl, propyl, isopropyl, n-butyl, isobutyl, pentyl or hexyl, or an amino acid side chain residue, preferably a side chain residue of asparagine, cystein, aspartic acid, glutamine, glutamic acid, phenylalanine, histidine, isoleucine, lysine, leucine, methionine, proline, arginine, serine, threonine, tryptophane, valine and tyrosine.

In a preferred embodiment of the method of the present invention the orthogonally protected bifunctional amino acid is an N-alkyl amino acid. If the N-residue in an amino acid according to formulas (I), (II) or (III) is alkyl substituted the residues $R^3$ and $R^4$ are more likely to be oriented in a cis-orientation and, thus, will more readily form a cyclic peptide compound upon cyclization in a subsequent step. Thus, in a particular preferred embodiment $R^1$ is a branched or linear $C_1$-$C_6$ alkyl, e.g. methyl, ethyl, propyl, isopropyl, N-butyl, isobutyl, pentyl or hexyl, or a branched or linear substituted $C_1$-$C_6$ alkyl, e.g. a substituted methyl, ethyl, propyl, iso-propyl, n-butyl, isobutyl, pentyl or hexyl.

In a further preferred embodiment $R^2$ is a branched or linear $C_1$-$C_6$ alkyl, e.g. methyl, ethyl, propyl, isopropyl, n-butyl, isobutyl, pentyl or hexyl, a branched or linear substituted, e.g. substituted methyl, ethyl, propyl, isopropyl, n-butyl, isobutyl, pentyl or hexyl, or $-CH_2-(CHY)_n-W-(CHY)_{n'}-X$. In this context $R^5$ is preferably hydrogen or an amino acid side chain residue. In an even more preferred embodiment of the method of the present invention $R^1$ is branched or linear $C_1$-$C_6$ alkyl, e.g. methyl, ethyl, propyl, isopropyl, n-butyl, isobutyl, pentyl or hexyl, or branched or linear substituted $C_1$-$C_6$ alkyl, e.g. substituted methyl, ethyl, propyl, isopropyl, n-butyl, isobutyl, pentyl or hexyl, and $R^2$ is $-CH_2-(CHY)_n-W-(CHY)_{n'}-X$. Again in this context it is preferred that $R^5$ is a hydrogen or an amino acid side chain residue.

In a preferred embodiment of the method of the present invention W is S, O or $N(CH_3)$. If W has the preferred meaning as indicated in the preceding sentence it is further preferred that $R^1$ has its preferred meaning, i.e. $R^1$ is branched or linear $C_1$-$C_6$ alkyl, e.g. methyl, ethyl, propyl, isopropyl, n-butyl, isobutyl, pentyl or hexyl, or branched or linear substituted $C_1$-$C_6$ alkyl, e.g. substituted methyl, ethyl, propyl, isopropyl, n-butyl, isobutyl, pentyl or hexyl. In an even more preferred embodiment W and $R^1$ have the preferred meaning outlined in this para. and $R^5$ is hydrogen or an amino acid side chain

residue.

The group Z within the orthogonally protected amino acid represents a bond, or a further component of or a part thereof, which will be present in the product of the synthesis employing the orthogonally protected amino acids of the present invention. Preferably this further component is already present in the orthogonally protected amino acid, when the synthesis of the first component is started. The first component will be attached to the amino and/or carboxy residue protected by $R^3$ and $R^4$, respectively. Thus, in a preferred embodiment Z can be any naturally or non-naturally occurring amino acid it is, however, even more preferred when Z is selected from the group consisting of alanine-A, asparagine-A, cystine-A, asparagine-A, aspartic acid-A, glutamine-A, glutamic acid-A, phenylalanine-A, glycine-A, histidine-A, isoleucine-A, lysine-A, leucine-A, methionine-A, proline-A, arginine-A, serine-A, threonine-A, tryptophane-A, valine-A, tyrosine-A, tert-butyl glycine-A, N-methyl phenylalanine-A, lysine(GlyMeDOTA)-A Hcy-A, Hhc-A, Pen-A, Aib-A, Nal-A, Aca-A, Ain-A, Hly-A, Achxa-A, Amf-A, Aec-A, Apc-A, Aes-A, Aps-A, Abu-A, Nva-A, FD-A, WD-A, YD-A, Cpa-A, Thp-A, D-Nal-A, Dpg-A, Dab-A, Nle-A, (N-$CH_3$)Cys-A, Orn-A, (N-$CH_3$)Hcy-A, (N-$CH_3$)Tyr-A, (N-$CH_3$)Tty-A, (N-$CH_3$) Tyr-A($CH_2$ $CH_2$ SH), Thr(OH)-A, Ser(ol)-A, Asp(ol)-A, Glu(ol)-A, Gln(ol)-A, Asn(ol)-A, Phe(4-F)-A, Phe(4-$NH_2$)-A, ε-Lys-A, δ-Orn-A, γ-Dab-A, β-Dap-A, wherein "A" is the amino or carboxyl group of the amino acid, a protected amino or carboxyl group or a direct or indirect bond to a surface. In order to prevent the modification of amino acid side chains during subsequent couplings/reactions involving the $R^3$ protected carboxyl residue and the $R^4$ protected amino residue the amino acids can optionally comprise (a) protected side chain residue(s). This residue will preferably not be cleaved under conditions that cleave $R^3$ and/or $R^4$.

In a further preferred embodiment Z can be a polypeptide as defined above. Again it is preferred that the terminal amino acid is linked via its amino and carboxy terminus, respectively, directly or indirectly, e.g. with an intermittent spacer, to a surface.

In a preferred embodiment the polypeptide is selected from the group consisting of a receptor ligand, an antibody, a single chain antibody or a binding fragment of an antibody or single chain antibody. The term antibody in this context has the meaning as defined above.

In a preferred embodiment the second component of the binding compound resulting from the synthesis employing the orthogonally protected amino acid is capable of chelating metals, in particular metal ions. A large variety of such metal chelating moieties are known in the art and are described, for example, in US 5,654,272, US 5,681,541, US 5,788,960, US 5,811,394, US 5,720,934, US 5,776,428, US 5,780,007, US 5,922,303, US 6,093,383, US 6,086,849, US 5,965,107, US 5,300,278, US 5,350,837, US 5,589,576, US 5,679,778 and US 5,879,659. The respectively described metal chelating residues are specifically referenced herewith and can all equally be used as metal chelating residues. It should also be pointed out that some metal chelating residues can also be considered polypeptides as defined above and, thus, the term chelating residues overlaps with the term "polypeptides" in as far as the polypeptide have the capability to chelat metal.

In a preferred method the metal chelating residue is selected from the group of preferred metal chelating residues indicated above under a) to 1). The chelating and in particular the preferred chelating moieties can optionally comprise one or more protected side chain residues or functions. The side chains or functions are protected to assure that the chelating moieties are not altered during coupling reactions taking place at the carboxy residue protected by $R^3$ and/or at the amino residue protected by $R^4$.

In a particular preferred embodiment of the method of the present invention the metal chelating residue is selected from the group consisting of:

a) -βDap-Phe-Cys-Thr-Ser-A;
b) -βDap-Tyr-Cys-Thr(ol)-A;
c) -βDap-Phe(4-F)-Cys-Thr(ol)-A;
d) -βDap-Phe(4-$NH_2$)-Cys-Thr-Ser-A;
e) -βDap-Dab-Cys-Thr-A;
f) -βDap-Phe(4-NH2)-Cys-Thr-A;
g) -βDap-Phe(4-NH2)-Cys-Thr(ol)-A;
h) -βDap-His-Cys-Thr(ol)-A;
i) -βDap-Arg-Cys-Thr(ol)-A;
j) -βDap-Gly-Cys-Lys-$NH_2$-A;
k) -βDap-Ser-Cys-Thr(ol)-A;
l) -βDap-Dab-Cys-Thr(ol)-A;
m) -βDap-Gly-Cys-Thr(ol)-A;
n) -βDap-Dab-Cys-Ser(ol)-A;
o) -βDap-Ser-Cys-Thr-NH($CH_2CH_2O$)$_2$ $CH_2CH_2$NH-A;
p) -βDap-Orn-Cys-Thr(ol)-A
q) -βDap-Dap-Cys-Thr(ol)-A;

r) -βDap-Lys-Cys-Thr(ol)-A; and
s) -βDap-Lys-Cys-NH-A;

Again the preferred chelating moieties can optionally comprise one or more protected side chain residues and "A" has the meaning as outlined above, preferably it means a direct or indirect bond to a surface.

For diagnostic purposes it is also possible to include a dye as a second component in the amino acid used in the method of the present invention.. The dye can be any of the dyes mentioned above and particularly preferred dyes are fluorescent dyes. The skilled person is aware of a large number of dyes, which are similarly suitable for imaging purposes, in particular for *in vivo* imaging purposes, which include, for example, fluorescent dyes as described in WO 00/61194, WO 00/71162, WO 01/52746, WO 01/52743 and WO 01/62156 and which can all be part of the amino acid employed in the method of the invention.

For therapeutic purposes the amino acid employed in the method of the present invention can also comprise a therapeutic agent as outlined above. This agent can be any therapeutic agent and preferably includes, therapeutic agents which benefit from targeted delivery like, e.g. analgesics; antirheumatics; anthelminthics; antiallergics; antianemics; antiarrhythmics; antibiotics; angiogenesis inhibitors; antiinfectives; antidemenics (nootropics); antidiabetics; antidotes; antiemetics; antivertiginosics; antiepileptics; antihemorrhagics; antihypertonics; antihypotonics; anticoagulants; antimycotics; antitussive agents; antiviral agents; beta-receptor and calcium channel antagonists; broncholytic and antiasthmatic agent; chemokines; cytokines, in particular immune modulatory cytokines; mitogens; cytostatics; cytotoxic agents and prodrugs thereof; dermatics; hypnotics and sedatives; immunosuppressants; immunostimulants in particular activators of NF-κB, MAP kinases, STAT proteins and/or protein kinase B/Akt; peptide or protein drugs; in particular hormones and physiological or pharmacological inhibitors of mitogens, chemokines, or cytokines or their respective prodrugs. In a preferred embodiment the drug is selected from the group consisting of chemokines, cytokines, mitogens, cytostatics, cytotoxic agents and prodrugs thereof, immunostimulants, peptide or protein drugs, in particular hormones and physiological or pharmacological inhibitors of mitogens, chemokines, or cytokines or their respective prodrugs.

Preferred cytostatic or cytotoxic drug are alkylating substances, anti-metabolites, antibiotics, epothilones, nuclear receptor agonists and antagonists, anti-androgens, anti-estrogens, platinum compounds, hormones and antihormones, interferons and inhibitors of cell cycle-dependent protein kinases (CDKs), inhibitors of cyclooxygenases and/or lipoxygenases, biogenic fatty acids and fatty acid derivatives, including prostanoids and leukotrienes, inhibitors of protein kinases, inhibitors of protein phosphatases, inhibitors of lipid kinases, platinum coordination complexes, ethyleneimenes, methylmelamines, trazines, vinca alkaloids, pyrimidine analogs, purine analogs, alkylsulfonates, folic acid analogs, anthracendiones, substituted urea, methylhydrazin derivatives. Cytostatic or cytotoxic drugs include without limitation acediasulfone, aclarubicine, ambazone, aminoglutethimide, L-asparaginase, azathioprine, bleomycin, busulfan, calcium folinate, carboplatin, carpecitabine, carmustine, celecoxib, chlorambucil, cis-platin, cladribine, cyclophosphamide, cytarabine, dacarbazine, dactinomycin dapsone, daunorubicin, dibrompropamidine, diethylstilbestrole, docetaxel, doxorubicin, enediynes, epirubicin, epothilone B, epothilone D, estramucin phosphate, estrogen, ethinylestradiole, etoposide, flavopiridol, floxuridine, fludarabine, fluorouracil, fluoxymesterone, flutamide fosfestrol, furazolidone, gemcitabine, gonadotropin releasing hormone analog, hexamethylmelamine, hydroxycarbamide, hydroxymethylnitrofurantoin, hydroxyprogesteronecaproat, hydroxyurea, idarubicin, idoxuridine, ifosfamide, interferon α, irinotecan, leuprolide, lomustine, lurtotecan, mafenide sulfate olamide, mechlorethamine, medroxyprogesterone acetate, megastrolacetate, melphalan, mepacrine, mercaptopurine, methotrexate, metronidazole, mitomycin C, mitopodozide, mitotane, mitoxantrone, mithramycin, nalidixic acid, nifuratel, nifuroxazide, nifuralazine, nifurtimox, nimustine, ninorazole, nitrofurantoin, nitrogen mustards, oleomucin, oxolinic acid, pentamidine, pentostatin, phenazopyridine, phthalylsulfathiazole, pipobroman, prednimustine, prednisone, preussin, procarbazine, pyrimethamine, raltitrexed, rapamycin, rofecoxib, rosiglitazone, salazosulfapyridine, scriflavinium chloride, semustine streptozocine, sulfacarbamide, sulfacetamide, sulfachlopyridazine, sulfadiazine, sulfadicramide, sulfadimethoxine, sulfaethidole, sulfafurazole, sulfaguanidine, sulfaguanole, sulfamethizole, sulfamethoxazole, co-trimoxazole, sulfamethoxydiazine, sulfamethoxypyridazine, sulfamoxole, sulfanilamide, sulfaperin, sulfaphenazole, sulfathiazole, sulfisomidine, staurosporin, tamoxifen, taxol, teniposide, tertiposide, testolactone, testosteronpropionate, thioguanine, thiotepa, tinidazole, topotecan, triaziquone, treosulfan, trimethoprim, trofosfamide, UCN-01, vinblastine, vincristine, vindesine, vinblastine, vinorelbine, and zorubicin, or derivatives or analogs thereof.

As the orthogonally protected amino acids can form the starting point for the synthesis of binding compounds according to the method of the present invention the first component, i.e. the binding component, is added to the carboxy residue protected by $R^3$ and/or the amino residue protected by $R^4$. Thus, in order to add, e.g. a monomeric building block to either $R^3$ or $R^4$ the protective groups $R^3$ and/or $R^4$ have to be removed. If it is desired that the addition of this new monomeric building block is restricted to the carboxy and/or amino residue it is preferred that additional protection groups are not removed under conditions removing $R^3$ and/or $R^4$. Consequently, in a preferred embodiment, when Z is an amino acid residue, a polypeptide residue or a chelating residue, the amino acid residue, the polypeptide or the metal chelating residue carries one or more protection group(s), which (is) are stable under conditions that remove $R_3$ and/or $R_4$.

In a further preferred embodiment of the method of the present invention n is 1-3, e.g. 1, 2 or 3 and n' is 1-3,

e.g. 1, 2 or 3.

It the method of the present invention it is required to selectively remove the protective groups $R^3$ and/or $R^4$ in order to allow the addition of monomeric building blocks to the carboxy and/or amino residue. In order to allow directed addition to either the carboxy or the amino residue it is preferred that both residues are protected by different protective groups, which differ in the conditions required for their removal and which, thus, allow the specific removal of $R^3$ or $R^4$ without removing the respective other protective group. The skilled artisan is aware of a large variety of protective groups, which can be employed in organic synthesis. Protective groups (also called protecting groups) are reviewed in, for example, Wuts, M. and Greene, T.W. (supra) and Kocienski, P.J. (supra).

In a preferred embodiment of the method of the present invention, $R^3$ and $R^4$ are each selected from a different group of protective groups selected from the group of protective groups removable by a nucleophile, by acidic conditions, preferably under which the peptide is still bound to the resin, by hydrogenolysis, by mild base or by photolytic conditions.

Particularly preferred protective groups, which can be used in the method of the present invention are

(i) a protective group removed at acidic conditions, preferably at a pH between 4 and 6, which is selected from the group consisting of Boc or Trityl protecting groups;
(ii) a protective group removed by a nucleophile, which is selected from the group consisting of Fmoc or Dde protecting groups;
(iii) a protective group removed by hydrogenolysis consisting of the allyl type, the tert-butyl type, the benzyl type or Dmab (4,4-dimethyl-2,6-dicyclohexylidene)-3-methylbutyl]-amino}benzyl ester;
(iv) a protective group removed by radiation, which is selected from the group consisting of nitroveratryloxy carbonyl, nitrobenzyloxy carbonyl, dimethyl dimethoxybenzyloxy carbonyl, 5-bromo-7-nitroindolinyl, o-hydroxy-$\alpha$-methyl cinnamoyl, and 2-oxymethylene anthraquinone.

Particular combinations of protective groups for $R^3$ and $R^4$ are preferred. It is preferred that $R^3$ protecting the carboxy group is removable by hydrogenolysis, mild base or photolytic conditions and $R^4$ protecting the amino group is removable by a nucleophile or acidic conditions, preferably under conditions which allow the peptide to still be bound to the resin. Examples of such preferred combinations include protective groups removed by hydrogenolysis and by a nucleophile. In a particular preferred embodiment $R^3$ is removed by hydrogenolysis and $R^4$ is removed by a nucleophile.

In an even more preferred embodiment of the method of the present invention $R^3$ is selected from the group of protective groups consisting of a protective group of the allyl type, the tert-butyl type and the benzyl type and $R^4$ is selected from the group of protective groups consisting of Fmoc, Boc and Dde.

Although the orthogonally protected bifunctional amino acid employed in the method of the present invention can exhibit any stereoisomery it is preferred that $R^2$ has an L configuration.

In a particular embodiment of the method of the present invention the starting orthogonally protected bifunctional amino acid of the present invention has the formula (XIII):

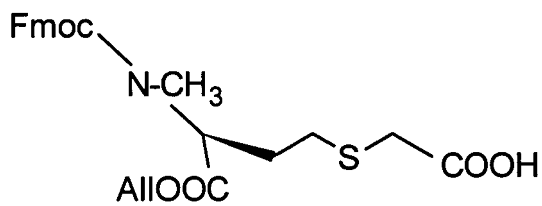

(XIII)

or is linked via the free carboxy terminus either directly or indirectly to a polypeptide, a dye, a therapeutic agent, a metal chelating agent or a surface.

Methods for making the orthogonally protected bifunctional amino acids, which can be employed in the method of the present invention are known to the skilled person and/or would be apparent to someone of skill based on the teaching contained herein. In particular, if $R^1$ or $R^2$ have the meaning $-CH_2-(CHY)_n-W-(CHY)_{n'}-X$, and Y has the meaning COZ, NHZ or Z and wherein Z is an amino acid, a polypeptide, a direct or indirect bond to a metal chelating residue, a dye, a therapeutic compound or a surface. The polypeptide, the metal chelating residue, the dye, or the chemotherapeutic compound can be synthesized independent from the remaining compound and might only be linked to the amino acid residue after completion of the first component. However, it is preferred that the second component or at least part of

the second component is already present in the amino acid employed in the method of the present invention.

Methods for synthesizing polypeptides are well known in the art and are routinely carried out on solid phase matrices. Similarly methods for making metal chelating residues are disclosed in, e.g. US 5,443,815; US 5,807,537; US 5,814,297; US 5,866,097; US 5,997,844; US 6,074,627; WO 95/31221 and WO 95/33497. If the second component, i.e. Z, is a polypeptide the linkage to the amino acid of the present invention can be accomplished via peptide bonds. This similarly applies to metal chelators of the polypeptide type.

However, in general metal chelators, dyes, therapeutic compounds or surfaces may be linked to the amino acid of the present invention via any suitable residue including carbon, nitrogen, sulphur or oxygen residues.

After the selective deprotection of $R^3$ and/or $R^4$, preferably of $R^3$ or $R^4$, the amino acid is capable of undergoing a coupling reaction involving either the free amino and/or carboxy groups. Thus, as a further step the method of the present invention comprises the step of:

(ii) coupling a monomeric building block to the deprotected carboxy or amino group of the amino acid.

In some cases it might be possible to simultaneously couple a monomeric building block, which comprises, for example, an activated amino and an activated carboxy function in a single directional step to both the amino and the carboxy group of the amino acid of the present invention. In these cases it might be necessary to remove both $R^3$ and $R^4$ simultaneously. The monomeric building block can be any chemical residue capable of reacting with the deprotected carboxyl or amino function of the amino acid of the present invention. The monomeric building block itself can comprise one or more different monomers, i.e. it can itself be a dimer, trimere or multimere, which is, however, added as a single "momomeric" block. In a preferred embodiment the monomeric building block is selected from the group consisting of alanine, asparagine, cystine, asparagine, aspartic acid, glutamine, glutamic acid, phenylalanine, glycine, histidine, isoleucine, lysine, leucine, methionine, proline, arginine, serine, threonine, tryptophane, valine, tyrosine, tert-butyl glycine, N-methyl phenylalanine, lysine(GlyMeDOTA) Hcy, Hhc, Pen, Aib, Nal, Aca, Ain, Hly, Achxa, Amf, Aec, Apc, Aes, Aps, Abu, Nva, FD, WD, YD, Cpa, Thp, D-Nal, Dpg, Nle, $(N\text{-}CH_3)$Cys, $(N\text{-}CH_3)$Hcy, $(N\text{-}CH_3)$Tyr, $(N\text{-}CH_3)$Tty, $(N\text{-}CH_3)$Tyr $(CH_2\ CH_2\ SH)$, Thr(OH), Ser(ol), Asp(ol), Glu(ol), Gln(ol), Asn(ol), Phe(4-F), Phe(4-$NH_2$), ε-Lys, δ-Orn, γ-Dab, β-Dap, a di, tri, tetra or pentapeptide comprising any combinations of above amino acids, a polypeptide and a ligand. Preferably the ligand is selected from the group consisting of an antibody, a single chain antibody, a binding fragment of an antibody or single chain antibody and a peptide ligand. Ligands are capable to bind to, e.g. surface structures of cells or connective tissue.

In most embodiments of the method of the present invention it will be required that more than one momomeric building block is subsequently added to either the carboxy or amino terminus. The coupling of the monomeric building block to the carboxy or amino function can be via any group capable to react with either of these functions. Preferably an activated amino group is coupled to the carboxy function and an activated carboxy group is coupled to the amino function. To allow further couplings of momomeric building blocks the monomeric building block coupled in the first coupling reaction as well as in later coupling reactions preferably comprises (a) protective group(s) $R^3$ and/or $R^4$ and optionally one or more protective group(s) which is (are) stable under conditions that remove $R^3$ and/or R4. The groups $R^3$ and $R^4$ have the same orthogonal properties as outlined above. However, it is possible that $R^3$ and/or $R^4$ of the monomeric building block protect other functionalities than an amino or carboxyl group, including e.g. hydroxy, aldehyde, keto, thio group and the like. It will be apparent to someone of skill in the art which protective groups will provide appropriate protection of one of these other functionalities while maintaining orthogonallity with respect to the respective other protective group.

Accordingly the method of the present invention can comprise in a preferred embodiment the further steps of:

(iii) selectively removing the protective group $R^3$ or $R^4$ from the monomeric building block or the amino acid, and
(iv) coupling a further monomeric building block, optionally comprising (a) protective group(s) $R^3$ and/or $R^4$ to the deprotected monomeric building block or amino acid.

In cases where, for example, the protective group $R^3$ was first removed from the amino acid and the monomeric building block was coupled to the free carboxy residue it is possible to add a further monomeric building block to the amino function of the amino acid, which would require removal of $R^4$ from the amino acid or the monomeric building block can be added to the first monomeric building block. In this case the first monomeric building block preferably comprises a protective group $R^3$, which is orthogonal to $R^4$. It is then possible to add further monomeric building blocks to the first monomeric building block or alternate between the two growing chains as required.

In a preferred embodiment the synthesis of the first component is not completed after the addition of two monomeric building blocks but rather 3, 4, 5, 6, 7, 8, 9, 10, 11, 12 or more monomeric building blocks are added in total. Consequently, in a preferred embodiment of the method of the present invention the steps (iii) and (iv) are repeated one or more times, preferably four times leading to a hexapeptide, optionally after the last coupling step (iv) step (iii) is carried

out once and/or a cyclization reaction is carried out. By carrying out step (iii) after the final coupling step it is possible to remove $R^3$ and/or $R^4$, which might be present on the monomeric building block(s). If desired it is possible to include a further step in order to remove other protective groups, which might serve to protect side chain residues. Such a step can be included after addition of the last momomeric building block, after removal of $R^3$ and/or $R^4$ or after cyclization. In a preferred embodiment the monomeric building blocks added to the amino acid of the present invention are cyclized, preferably to form a cyclic peptide chain. Such cyclic peptide chains can serve as specific binding components within the binding compound synthesized according to the method of the present invention.

As has been described above in one embodiment of the invention it is possible to couple a monomeric building block firstly to either the amino or the carboxy residue. This reactions will result in an amino acid of the present invention carrying a monomeric building block both at its carboxy and amino terminus. Thus, in one embodiment two monomeric building blocks, optionally comprising (a) protective group(s) $R^3$ and/or $R^4$, are added subsequently or simultaneously, preferably subsequently, to both the deprotected carboxy and to the deprotected amino group of the amino acid. It was surprisingly found that the coupling of two monomeric building blocks to the carboxy and amino residue, respectively, led to less side reactions in subsequent coupling steps and consequently to higher yields if compared to coupling reactions carried out just on one terminus, e.g. if five monomeric building blocks are added subsequently to the carboxy terminus prior to cyclization.

In case that a monomeric building block has been added both to the amino and the carboxy terminus of the amino acid of the invention it is preferred that the method comprises the further steps of:

(v) selectively removing the protective group $R^3$ and/or $R^4$ from one of the monomeric building blocks, and
(vi) coupling a further monomeric building block, optionally comprising (a) protective group(s) $R^3$ and/or $R^4$ to the deprotected monomeric building block.

Again as outlined above it is preferred that once a third monomeric building block has been added, i.e. one to the carboxy and one to the amino terminus of the amino acid of the invention and one to either the amino terminal or carboxy terminal monomeric building block, that steps (v) and (vi) are repeated one or more times, and that optionally after the last coupling step (vi) step (v) is carried out once and/or a cyclisation reaction is carried out. The steps (v) and (vi) can be repeated for 1, 2, 3, 4, 5, 6, 7, 8, 9, 10, 11, 12 or more times, preferably for 2 more times, leading to a hexapeptide. It is particularly preferred that a polypeptide generated by the coupling reaction is cyclized and comprises altogether (including the amino acid of the present invention) six amino acid residues.

In a particular preferred embodiment the method of the present invention, comprises the following steps: removing $R^4$, coupling Phe-$R^4$, removing $R^3$, coupling Tyr-$R^3$, removing $R^4$, coupling Thr-$R^4$, removing $R^4$, coupling Lys-$R^4$, removing $R^4$, coupling Trp-$R^4$, removing $R^3$ and $R^4$, cyclisation and optionally cleavage from a surface and/or removing one or more protective group(s), which is (are) stable under conditions that remove $R^3$ and/or $R^4$.

In a preferred method of the present invention one or more monomeric building blocks are coupled to produce a cyclic peptide with the sequence according to formula (XIII):

cyclo[$X^3$-DTrp-Lys-$X^4$-$X^5$-$X^6$] (XIII),

wherein

$x^3$ is diphenyl-Ala, (1)Nal, (2)Nal, (4)Pal, Phe(4-F), Thioproline, Trp or Tyr;
$X^4$ is βAla(cyclopropyl), diaminopropanoic acid, Thr or Val;
$x^5$ is an amino containing a side-chain as either the D or L isomer, capable of conjugating to a metal chelating residue, a dye, or a chemotherapeutic compound, or a natural or unnatural α-amino acid, or a N- alkyl α-amino acid;
$X^6$ is a radical of an amino acid according to formula (I), (II) or (III).

Preferably in this structure the carboxy residue $X^6$ is connected to the amino residue of $X^3$ to form a peptide bond and conversely the amino residue of $X^6$ is connected to the carboxy residue of $X^5$ to form a peptide bond.

In a particular preferred method of the present invention one or more monomeric building blocks are coupled to produce a cyclic peptide with the sequence:

a) cyclo[Tyr-DTrp-Lys-Thr-Phe-(NMe)hCys];
b) cyclo[lNa1-DTrp-Lys-Thr-Met-(NMe)Phe];
c) cyclo[Trp-DTrp-Lys-Thr-Met-(NMe)Phe];
d) cyclo[lNa1-DTrp-Lys-Val-Met-(NMe)Phe];
e) cyclo[Phe(4-F)-DTrp-Lys-Thr-Met-(NMe)Phe];
f) cyclo[Tyr-DTrp-Lys-Val-Met-(NMe)Phe];

g) cyclo[1Nal-DTrp-Lys-Thr-Lys(GlyMeDOTA)-(NMe)Phe];
h) cyclo[Tyr-DTrp-Lys-Thr-Met-(NMe)Phe];
i) cyclo[2Nal-DTrp-Lys-Thr-Met-(NMe)Phe];
j) cyclo[Tyr-DTrp-Lys-Thr-Met-Tpi];
k) cyclo[Tyr-DTrp-Lys-BAla(cyclopropyl)-Met-(NMe)Phe];
l) cyclo[Tyr-DTrp-Lys-Dpr-Met-(NMe)Phe];
m) cyclo[ThioPro-DTrp-Lys-Thr-Met-Phe];
n) cyclo[DiphenylAla-DTrp-Lys-Thr-Met-(NMe)Phe];
o) cyclo[(4)Pal-DTrp-Lys-Thr-Met-(NMe)Phe].

These structures have somatostatin receptor binding capacity. Preferably in these structures the amino group of the left most amino acid forms a peptide bond with carboxy group of the right most amino acid.

As has been pointed out above it is preferred that the amino acid employed in the method of the present invention comprises a metal chelating residue. It is particularly preferred if such a metal chelating residue is present when the first component is capable of specific receptor binding, in particular binding of the somatostatin receptor. Such a binding compound can be used to recruit diagnostic or therapeutic metals, in particular metal ions, to the diseased area, tissue or cells. A large number of metals, which can serve either therapeutic or diagnostic purposes, e.g. for radiation therapy or as contrast agent, are known in the art. Thus, in a further embodiment of the method of the present invention, wherein the synthesized compound comprises a metal chelating residue the compound is radiolabled with a metal. Optionally the method comprises additional purification steps prior and/or after radiolabeling. In a particular preferred embodiment the method of the present invention comprises the steps of:

(vii) optionally purifying the binding compound and
(viii) radiolabeling the binding compound with $^{186}$Re, $^{188}$Re, $^{212}$Bi, $^{213}$Bi, $^{90}$Y, $^{153}$Sm, $^{47}$Sc, $^{68}$Ga, $^{94m}$Tc, $^{99m}$Tc, $^{67}$Cu, $^{166}$Ho, $^{223}$Ra, $^{225}$Ac, $^{18}$F, $^{125}$I, $^{131}$I, $^{123}$I, or $^{211}$At or a salt thereof. Optionally in a further step the radiolabeled binding compound is purified.

To administer the binding compound or the radiolabeled binding compound produced according to the method of the present invention it is preferred that the method, further comprises the steps of:

(ix) optionally purifying the binding compound and
(x) admixing the binding compound with a pharmaceutically acceptable carrier, additive(s), and/or buffer.

Suitable buffers are all physiologically acceptable buffers as long as they do not conflict with the binding compound and include without limitation phosphate buffered saline, Hepes, Tris or the like, preferably with a physiological amount of salt, e.g. sodium chloride. Additives include, for example, preservatives, sugars, e.g. glucose, sorbitol, sucrose, maltose, trehalose, lactose, dextran or raffinose, or antioxidants, e.g. $\alpha$-tocopherol.

The binding compounds produced according to the method of the present invention have the capability to bind to structures which are present in or in the vicinity of diseased tissue or cells and accordingly they can be used to target the dyes, metal ions, therapeutic compounds etc. which are part of the binding compound to the respective site of the disease. Thus, a further aspect of the present invention is the use of a binding compound producible according to the method of the present invention, for the production of a therapeutic or diagnostic for the treatment or diagnosis of a proliferative diseases, infectious diseases, vascular diseases, rheumatoid diseases, inflammatory diseases, immune diseases, in particular autoimmune diseases and allergies.

In a preferred embodiment the proliferative diseases includes but are not limited to malignomas (e.g., carcinomas, sarcomas) of the gastrointestinal or colorectal tract, liver, pancreas, kidney, bladder, thyroid, prostate, endometrium, ovary, testes, melanoma, dysplastic oral mucosa, invasive oral cancers, small cell and non-small cell lung carcinomas; mammary tumors, e.g. a hormone-dependent breast cancers, hormone independent breast cancers; transitional and squamous cell cancers; neurological malignancies including neuroblastoma, gliomas, astrocytomas, osteosarcomas, meningiomas; soft tissue sarcomas; hemangioamas and endocrinological tumors, e.g. pituitary adenomas, pheochromocytomas, paragangliomas, haematological malignancies including lymphomas and leukemia. Because of the expression of somatostatin receptor the treatment and diagnosis of the following tumors is particularly preferred: neuroendocrine tumors such as pituitary adenomas, pheochromocytomas, paragangliomas, medulary thyroid carcinomas, small cell lung cancers. neurological malignanciessuch as astrocytomas, meningiomas, human breast tumors, malignant lymphomas, renal cell carcinomas and prostate tumors.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

In the foregoing and in the following examples, all temperatures are set forth uncorrected in degrees Celsius, and all parts and percentages are by weight, unless otherwise indicated. The entire disclosure[s] of all applications, patents and publications, cited herein are incorporated by reference herein.

The following examples can be repeated with similar success by substituting the generically or specifically described reactants and/or operating conditions of this invention for those used in the preceding examples.

From the foregoing description, one skilled in the art can easily ascertain the essential characteristics of this invention and, without departing from the spirit and scope thereof, can make various changes and modifications of the invention to adapt it to various usages and conditions.

## Brief Description of the Figure

**Fig. 1** Reaction schema leading to Fmoc-NMeHcy(CH2CO2H)-Oallyl starting from N-Boc methionine

## Example

### Synthesis of Fmoc-NMeHcy($CH_2CO_2H$)-Oallyl (XIII)

### Intermediate 2

To a 3-neck, 2-L round-bottomed flask equipped with heating mantle, reflux condenser, $Na_2SO_4$ drying tube and thermometer, was added N-Boc methionine, paraformaldehyde, and $MgSO_4$ in 1 L of toluene. To this was added pTsOH-$H_2O$, and the mixture was heated with stirring to an internal temperature of ~90°C for approximately 3 hours. A white precipitate developed on the inside of the reflux condenser.

The reaction was cooled to rt then placed in an ice/water bath. 800 mL of saturated $NaHCO_3$ was added with the evolution of $CO_2$. A thick yellow sludge developed, and it was necessary to use mechanical stirring to mix the solution. The entire mixture was filtered through Whatman 1 filter paper in a large Buchner funnel under aspirator pressure. The solid residue was washed with ~400 mL of EtOAc, and the combined filtrate was transferred to a 2-L separatory funnel. The organic layer was separated and washed with ~300 mL of water. The organic layer was then dried over solid $Na_2SO_4$ and concentrated

The crude product was dissolved in 2:1 Hexanes/EtOAc and eluted through 150 g of flash silica-gel. A band of orange color was retained on the column, and the product was collected in three large fractions. The fractions were combined and concentrated to a light yellow oil. 98.48g of pure product 2 was obtained (94% yield; theoretical yield = 104.92g)

### Intermediate 3

To a 500-mL single-neck round-bottomed flask was added oxazolidinone **2** in 65 mL of $CH_2Cl_2$. The flask was placed in an ice/water bath, a magnetic stir bar was added, and a 125-mL pressure-equalized addition funnel was attached. In a separate 250-mL erlenmeyer flask, the TFA and TES were combined in 40 mL of $CH_2Cl_2$. The TES and TFA are not miscible by themselves. The TFA/TES solution was added to the addition funnel, and then added dropwise to the oxazolidinone solution at 0°C. The reaction continued to stir for approximately 3 hours as the ice/water bath slowly warmed to rt. The reaction solution was concentrated by rotary evaporation, and the residue was chased three times with $CH_2Cl_2$. The residue was then taken up in 100 mL of water and extracted with t-butyl methyl ether (TBME) (3 X 50 mL). The TBME extractions were orange-red in color. The aqueous layer was concentrated by rotary evaporation under high vacuum, and the residue was chased with EtOH (3 X 50 mL). A thick light yellow oil resulted. The oil was covered with 150 mL of TBME and stirred magnetically for several hours. A white solid formed which was collected by vacuum filtration. The solid was washed with TBME and dried under high vacuum. 9.44 grams of pure N-methylmethionine 3 were collected (75% yield; theoretical yield = 12.53g).

### Intermediate 4

To a 500-mL 3-neck roundbottomed flask, equipped with glass stoppers, stir bar, dry-ice condenser, and nitrogen bubbler, was added N-methylmethionine 3. The flask was placed in a dry-ice/acetone bath, and dry-ice/acetone was added to the condenser. The entire apparatus was flushed with nitrogen through the nitrogen bubbler. Anhydrous ammonia was pumped into the flask through one of the side necks using a hose adaptor. After approximately 150 mL of ammonia condensed, the ammonia inlet was removed and the flask was sealed with a glass stopper. The flask was

then removed from the dry-ice/acetone bath. Small pieces of sodium, rinsed in hexanes, were added to the reaction until a deep blue color persisted. The reaction stirred for an additional 45 minutes during which time the color remained deep blue.

The reaction was quenched with solid $NH_4Cl$ until the blue color dissipated. The condenser was removed and the ammonia was allowed to evaporate overnight. The crude white solid was taken up in 250 mL of water and pH-adjusted to 5-6 with 1 M HCl. The aqueous solution was then extracted with $Et_2O$ (2 X 50mL), shell-frozen in a dry-ice/acetone bath, and lyophilized to a white solid. 25.70g of white solid were obtained. The weight-percent of NMeHcy (4) in the crude product was calculated to be 42.7 wt% assuming a quantitative yield.

**Intermediate 5**

To a 500-mL single-neck round-bottomed flask, equipped with magnetic stir bar, was added crude N-methylhomocysteine (4) followed by 50 mL of methanol. Approximately 30 mL of water was added to completely dissolve the starting material. The pH of the solution was measured to be ~6 using pH test strips. Sodium methoxide was added, and the pH increased to 9-10. Tert-butyl bromoacetate was then added, and the homogeneous solution was allowed to stir overnight at rt under normal atmosphere.

Fmoc-OSu was added directly to the reaction at this time. Equal amounts of THF and water were then added until all reactants were solubilized. Approximately 600 mL of total solution resulted, and the reaction had to be transferred to a 1000-mL roundbottom. The pH was measured at 6-7. A 1M solution of $K_2CO_3$ (20 mL, 103 mol%) was added to adjust the pH to 9-10. The reaction was allowed to stir overnight at rt under normal atmosphere. The reaction was concentrated by rotary evaporation to remove most of the organic solvents, and a yellow precipitate developed in the remaining aqueous layer. The pH of the aqueous layer was adjusted to 3-4 with 0.5 M $KHSO_4$, and was then extracted with EtOAc (3 X 50 mL). The combined organic extracts were washed with brine and dried over $Na_2SO_4$ before concentrating to a thick orange oil/foam. 9.279g of crude product was recovered.

The crude product was purified by column chromatography. Approximately 200g of flash silica-gel was used in a 2-inch diameter column. The column was built and loaded in neat $CHCl_3$. The top spots were eluted in neat $CHCl_3$, and the product eluted in 1% MeOH in $CHCl_3$ (note that the $CHCl_3$ contained 0.75% EtOH as a stabilizer). 5.972g of pure 5 was recovered from the column (63% yield). An additional 1.729g of 5 containing a small amount of the high-Rf impurities was also recovered.

**Intermediate 6**

To a 200-mL single-neck round-bottomed flask, equipped with magnetic stir bar and nitrogen balloon, was added compound **5** in 75 mL of $CH_3CN$. $KHCO_3$ was added directly to this solution followed immediately by allyl bromide. The reaction stirred overnight at rt under nitrogen.

The reaction stirred for an additional overnight period, after which no change in TLC was observed. At this time an additional 1.260 mL of allyl bromide (14.48 mmol, 110 mol%) and 526 mg of $KHCO_3$ (5.25 mmol, 40 mol%) were added, and the reaction continued to stir for a third overnight period. TLC analysis showed the complete conversion of starting material.

The reaction was concentrated by rotary evaporation and the residue was partitioned between 100 mL each of EtOAc and water. The EtOAc layer was separated, and the aqueous layer was extracted with EtOAc. The combined organic layers were washed with brine, dried over $Na_2SO_4$, and concentrated to a light yellow oil (6.985g; 101% crude yield).

The crude product was purified by column chromatography. 170g of flash silica-gel were used. The column was built in 5% EtOAc in hexanes, and the crude product was loaded in $CHCl_3$. The column was eluted with 5% $\rightarrow$ 30% EtOAc in hexanes; pure product began eluting with 10% EtOAc in hexanes. 5.515g of pure allyl ester was collected (80% yield).

$^1H$ NMR of compound 6 in $CD_3OD$:

**Synthesis of Fmoc-NMeHcy($CH_2CO_2H$)-Oallyl (XIII)**

To a 250-mL single-neck roundbottom flask equipped with a magnetic stir bar, was added the t-butyl ester 6 in 30 mL of $CH_2Cl_2$. To this was added TFA, and the reaction was allowed to stir at rt for 2 hours.

The reaction was concentrated and chased 3 times with $CH_2Cl_2$. The crude product was purified by column chromatography (75g flash silica-gel; build and load in $CHCl_3$; elute with 1 $\rightarrow$ 2% MeOH in $CHCl_3$). 4.72g of pure product (XIII) were collected (96% yield).

## Claims

1. Orthogonally protected bifunctional amino acid and salts thereof having the formula (I), (II) or (III):

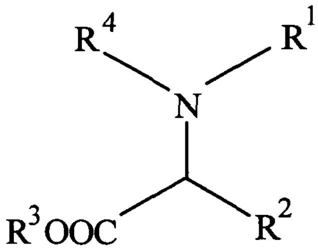

(I)

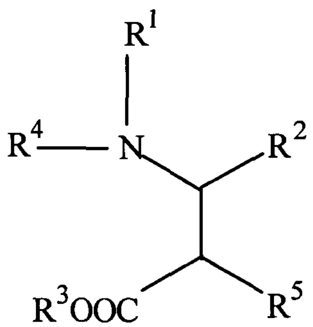

(II)

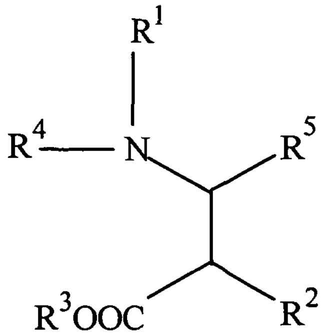

(III)

wherein,
$R^1$ and $R^2$ are independently of each other hydrogen, branched or linear $C_1$-$C_6$ alkyl, branched or linear substituted $C_1$-$C_6$ alkyl or $-CH_2-(CHY)_n-W-(CHY)_{n'}-X$, with the proviso that in formula I $R^1$ and $R^2$ are not hydrogen;
W is CHY, S, O, $N(CH_3)$, $N(C_2H_5)$ or $N(C_3H_7)$;
X is COOH, $NH_2$, COZ, NHZ or Z;
Y is for each CHY independently hydrogen, methyl or halogen;
Z is an amino acid residue; a polypeptide; a protective group, which can be selectively removed in the presence of $R^3$ and $R^4$; a direct or indirect link to a metal chelating residue, a dye, a therapeutic compound or a surface; or a bond,
n is 0-6;
n' is 1-6, or n' is 0-6 under the proviso that W is CHY;
$R^3$ is a protective group, which can be selectively removed in the presence of $R^4$;
$R^4$ is a protective group, which can be selectively removed in the presence of $R^3$; and
$R^5$ is hydrogen, branched or linear $C_1$-$C_6$ alkyl, branched or linear substituted $C_1$-$C_6$ alkyl or an amino acid side chain residue.

2. Orthogonally protected bifunctional amino acid according to claim 1, wherein $R^1$ is branched or linear $C_1$-$C_6$ alkyl or branched or linear substituted $C_1$-$C_6$ alkyl.

3. Orthogonally protected bifunctional amino acid according to claims 1 or 2, wherein $R^2$ is branched or linear $C_1$-$C_6$ alkyl, branched or linear substituted $C_1$-$C_6$ alkyl or $-CH_{2-}(CHY)_n-W-(CHY)_{n'}-X$.

4. Orthogonally protected bifunctional amino acid according to any one of claims 1 to 3, wherein $R^1$ is branched or linear $C_1$-$C_6$ alkyl or branched or linear substituted $C_1$-$C_6$ alkyl and $R^2$ is $-CH_2-(CHY)_n-W-(CHY)_{n'}-X$.

5. Orthogonally protected bifunctional amino acid according to any one of claims 1 to 4, wherein W is S, O or $N(CH_3)$.

6. Orthogonally protected bifunctional amino acid according to any one of claims 1 to 5, wherein the amino acid residue is selected from the group consisting of alanine-A, asparagine-A, cystine-A, asparagine-A, aspartic acid-A, glutamine-A, glutamic acid-A, phenylalanine-A, glycine-A, histidine-A, isoleucine-A, lysine-A, leucine-A, methionine-A, proline-A, arginine-A, serine-A, threonine-A, tryptophane-A, valine-A, tyrosine-A, tert-butyl glycine-A, N-methyl phenylalanine-A, lysine(GlyMeDOTA)-A Hcy-A, Hhc-A, Pen-A, Aib-A, Nal-A, Aca-A, Ain-A, Hly-A, Achxa-A, Amf-A, Aec-A, Apc-A, Aes-A, Aps-A, Abu-A, Nva-A, FD-A, WD-A, YD-A, Cpa-A, Thp-A, D-Nal-A, Dpg-A, Nle-A, $(N-CH_3)$Cys-A, $(N-CH_3)$Hcy-A, $(N-CH_3)$Tyr-A, $(N-CH_3)$Tty-A, $(N-CH_3)$Tyr-A($CH_2$ $CH_2$ SH), Thr(OH)-A, Ser(ol)-A, Asp(ol)-A, Glu (ol)-A, Gln(ol)-A, Asn(ol)-A, Phe(4-F)-A, Phe(4-$NH_2$)-A, ε-Lys-A, δ-Orn-A, γ-Dab-A, β-Dap-A, optionally comprising protected side chain residues,
wherein A is the amino or carboxyl group of the amino acid, a protected amino or carboxyl group or a direct or indirect link to a surface.

**7.** Orthogonally protected bifunctional amino acid according to any one of claims 1 to 5, wherein the polypeptide is selected from the group consisting of a receptor ligand, an antibody, a single chain antibody or a binding fragment of an antibody or single chain antibody.

**8.** Orthogonally protected bifunctional amino acid according to any one of claims 1 to 5, wherein the metal chelating residue is selected from the group consisting of

a) $C(pgp)^s$-(aa)-$C(pgp)^s$, wherein $(pgp)^s$ is hydrogen or a thiol protecting group and (aa) is any [alpha]- or [beta]-amino acid not comprising a thiol group;
b) a substance according to formula (IV) or (V)

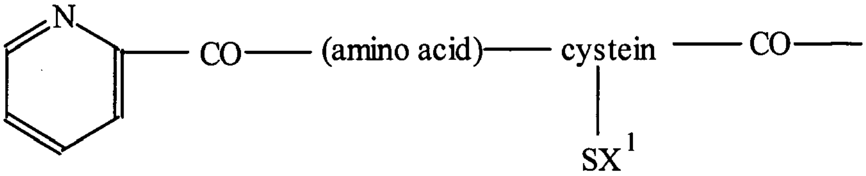

(IV)

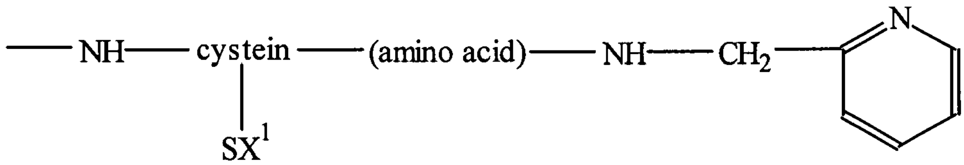

(V),

wherein $X^1$=H or a protecting group;
(amino acid)=any amino acid;
c) a substance according to formula (VI)

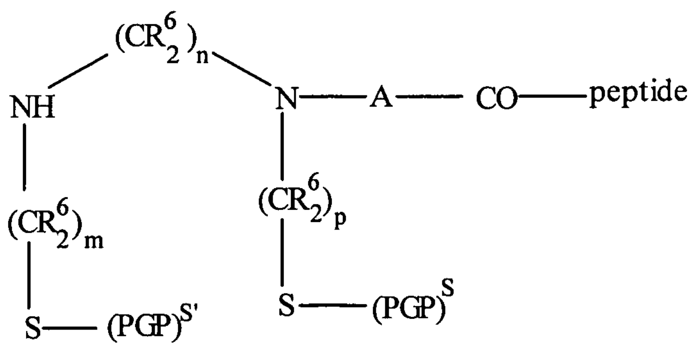

(VI),

wherein each $R^6$ is independently H, $CH_3$ or $C_2H_5$, each (pgp)' is independently a thiol protecting group or H; m, n and p are independently 2 or 3; A is linear $C_1$-$C_8$ alkyl, substituted linear $C_1$-$C_8$ alkyl, cyclic $C_3$-$C_8$ alkyl, substituted cyclic $C_3$-$C_8$ alkyl, aryl, substituted aryl, or a combination thereof; and
d) a substance according to formula (VII)

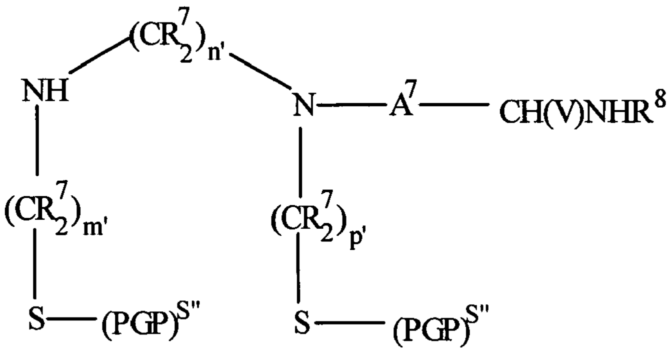

(VII),

wherein each $R^7$ is independently H, $CH_3$ or $C_2H_5$; each (pgp)S" is independently a thiol protecting group or H; m', n' and p' are independently 2 or 3; $A^1$ is linear $C_1$-$C_8$ alkyl, substituted linear $C_1$-$C_8$ alkyl, cyclic $C_3$-$C_8$ alkyl, substituted cyclic $C_3$-$C_8$ alkyl, aryl, substituted aryl, or a combination thereof; V is H or a COX; $R^8$ is H or a covalent link to X;

e) diethylenetriaminepentaacetic acid (DTPA);

f) a derivative of DTPA having a formula (VIII)

$$(HOOCCH_2)_2N(CR^9_2)(CR^9_2)N(CH_2COOH)(CR^9_2)(CR^9_2)N(CH_2COOH)_2$$

(VIII),

wherein each $R^9$ is independently H, $C_1$ to $C_4$ alkyl, or aryl and at least one $R^9$ is a covalent link to X;

g) ethylenediaminetetraacetic acid (EDTA);

h) a derivative of EDTA having a formula (IX)

$$(HOOCCH_2)_2N(CR^{10}_2)(CR^{10}_2)N(CH_2COOH)_2$$

(IX),

wherein each $R^{10}$ is independently H, $C_1$ to $C_4$ alkyl, or aryl and one $R^{10}$ is covalently linked to X;

i) 1,4,7,10-tetraazacyclododecanetetraacetic acid and derivatives thereof;

j) a substance according to formula (X)

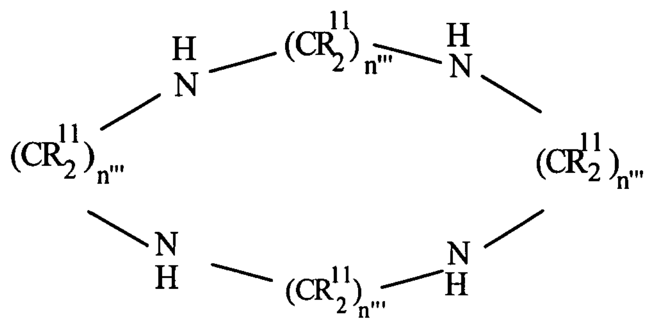

(X),

wherein n"' is an integer that is 2 or 3 and where each $R^{11}$ is independently H, $C_1$ to $C_4$ alkyl, or aryl and one $R^{11}$ is covalently linked to X;

k) a substance according to formula (XI) comprising a single thiol

$$A^3\text{-}CZ^3(B^3)\text{-}\{C(R^{12}R^{13})\}_{n''}\text{-}X^3 \qquad (XI),$$

wherein $A^3$ is H, HOOC-, $H_2NOC$-, -NHOC-, -OOC-, $R^{16}_2$ NOC-, X-NHOC-, X-OOC-, or $R^{15}$; $B^3$ is H, SH, $-NHR^{14}$, $-N(R^{14})$-, $X\text{-}NR^{14}$- or $R^{15}$; $Z^3$ is H or $R^{15}$; $X^3$ is SH, $-NHR^{14}$, $-N(R^{14})$-, $X\text{-}NR^{14}$- or $R^{15}$; $R^{12}$, $R^{13}$, $R^{14}$ and $R^{15}$ are independently H, straight chain $C_1$-$C_8$ alkyl, e.g. methyl, ethyl, propyl, isopropyl, n-butyl, isobutyl, pentyl, hexyl, heptyl or octyl, branched chain $C_1$-$C_8$ alkyl, or cyclic $C_3$-$C_8$ alkyl, e.g. propyl, butyl, pentyl, hexyl, heptyl or octyl; n" is 0, 1 or 2; $R^{16}$ is $C_1$-$C_4$ alkyl, an amino acid, or a peptide comprising 2 to about 10 amino acids; and: (1) where $B^3$ is $-NHR^{14}$, $X\text{-}NR^{14}$- or $-N(R^{14})$-, $X^3$ is SH and n" is 1 or 2; (2) where $X^3$ is - $NHR^{14}$, $X\text{-}NR^{14}$-, or $-N(R^{14})$-, $B^3$ is SH and n" is 1 or 2; (3) where $B^3$ is H or $R^{15}$, $A^3$ is HOOC-, $H_2NOC$-, X-NHOC-, X-OOC-, -NHOC-, or -OOC-, $X^3$ and n" is 0 or 1; (4) where $A^3$ is H or $R^{15}$, in cases where $B^3$ is SH, $X^3$ is $NHR^{14}$, $X\text{-}NR^{14}$-, or $-N(R^{14})$- and where $X^3$ is SH, $B^3$ is $-NHR^{14}$ $X\text{-}NR^{14}$- or $-N(R^{14})$ and n" is 1 or 2; (5) where $X^3$ is H or $R^{15}$, $A^3$ is HOOC-, $H_2NOC$-, -NHOC-, -OOC-, X-NHOC- or X-OOC- and $B^3$ is SH; (6) where $Z^3$ is methyl, $X^3$ is methyl, $A^3$ is HOOC-, $H_2NOC$-, -NHOC-, -OOC-, X-NHOC- or X-OOC- and $B^3$ is SH and n is 0; and (7) where $B^3$ is SH, $X^3$ is not SH and where $X^3$ is SH, $B^3$ is not SH, and

l) a substance according to formula (XII)

-βDap-Xaa-Cys-Zaa-A (XII),

wherein Xaa is an L-α-amino acid;

Zaa is an α-amino acid, an α-amino acid amide, an aminoethylether, a β-aminol, or a peptide containing from two to ten α-amino acids, said peptide having a carboxyl terminal α-amino acid, α-amino acid amide, aminoethylether, or β-aminol, and A is the amino or carboxyl group of the amino acid, a protected amino or carboxyl group or a direct or indirect link to a surface,

optionally comprising one or more protected side chain residues.

**9.** Orthogonally protected bifunctional amino acid according to claim 8, wherein the metal chelating residue is selected from the group consisting of:

a) -βDap-Phe-Cys-Thr-Ser-A;
b) -βDap-Tyr-Cys-Thr(ol)-A;
c) -βDap-Phe(4-F)-Cys-Thr(ol)-A;
d) -βDap-Phe(4-$NH_2$)-Cys-Thr-Ser-A;
e) -βDap-Dab-Cys-Thr-A;
f) -βDap-Phe(4-NH2)-Cys-Thr-A;

g) -βDap-Phe(4-NH2)-Cys-Thr(ol)-A;
h) -βDap-His-Cys-Thr(ol)-A;
i) -βDap-Arg-Cys-Thr(ol)-A;
j) -βDap-Gly-Cys-Lys-$NH_2$-A;
k) -βDap-Ser-Cys-Thr(ol)-A;
l) -βDap-Dab-Cys-Thr(ol)-A;
m) -βDap-Gly-Cys-Thr(ol)-A;
n) -βDap-Dab-Cys-Ser(ol)-A;
o) -βDap-Ser-Cys-Thr-NH($CH_2CH_2O)_2$ $CH_2CH_2$NH-A;
p) -βDap-Orn-Cys-Thr(ol)-A
q) -βDap-Dap-Cys-Thr(ol)-A;
r) -βDap-Lys-Cys-Thr(ol)-A; and
s) -βDap-Lys-Cys-NH-A;

optionally comprising one or more protected side chain residues.

**10.** Orthogonally protected bifunctional amino acid according to any one of claims 1 to 9, wherein the amino acid residue, the polypeptide or the metal chelating residue carries one or more protection group(s), which (is) are stable under conditions that remove $R_3$ and/or $R_4$.

**11.** Orthogonally protected bifunctional amino acid according to any one of claims 1 to 10, wherein n is 1-3 and n' is 1-3.

**12.** Orthogonally protected bifunctional amino acid according to any one of claims 1 to 11, wherein $R^3$ and $R^4$ are each selected from a different group of protective groups selected from a protective group removable by a nucleophile, by acidic conditions, by hydrogenolysis, by mild base or by photolytic conditions.

**13.** Orthogonally protected bifunctional amino acid according to claim 12, wherein

(i) a protective group removed at acidic conditions, preferably at a pH between 4 and 6, which is selected from the group consisting of Boc or Trityl protecting groups;
(ii) a protective group removed by a nucleophile, which is selected from the group consisting of Fmoc or Dde protecting groups;
(iii) a protective group removed by hydrogenolysis consisting of the allyl type, the tert-butyl type, the benzyl type or Dmab (4,4-dimethyl-2,6-dicyclohexylidene)-3-methylbutyl]-amino}benzyl ester;
(iv) a protective group removed by radiation, which is selected from the group consisting of nitroveratryloxy carbonyl, nitrobenzyloxy carbonyl, dimethyl dimethoxybenzyloxy carbonyl, 5-bromo-7-nitroindolinyl, o-hydroxy-α-methyl cinnamoyl, and 2-oxymethylene anthraquinone.

**14.** Orthogonally protected bifunctional amino acid according to one of claims 1 to 13, wherein $R^3$ is removed by hydrogenolysis, mild base or photolytic conditions and $R^4$ is removed by a nucleophile or acidic conditions.

**15.** Orthogonally protected bifunctional amino acid according to any one of claims 1 to 14, wherein $R^3$ is selected from the group of protective groups consisting of a protective group of the allyl type, the tert-butyl type and the benzyl type and $R^4$ is selected from the group of protective groups consisting of Fmoc, Boc and Dde.

**16.** Orthogonally protected bifunctional amino acid according to any one of claims 1 to 15, wherein $R^2$ has an L configuration.

**17.** Orthogonally protected bifunctional amino acid according to claim 1, having the formula (XIII):

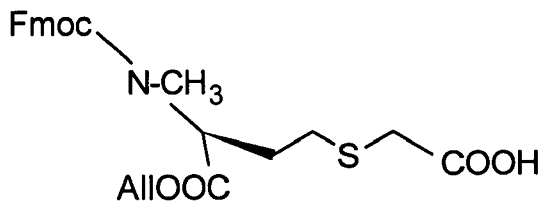

(XIII)

**18.** Method for producing orthogonally protected bifunctional amino acid according to any one of claims 1 to 16, comprising the step of reacting a compound of formula (XIV) to formula (XVI):

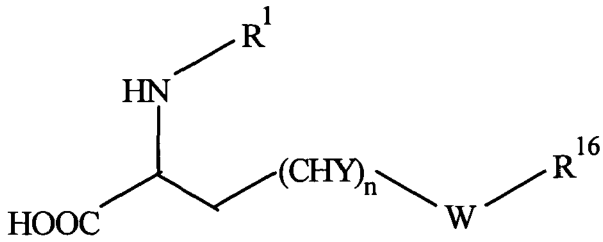

(XIV)

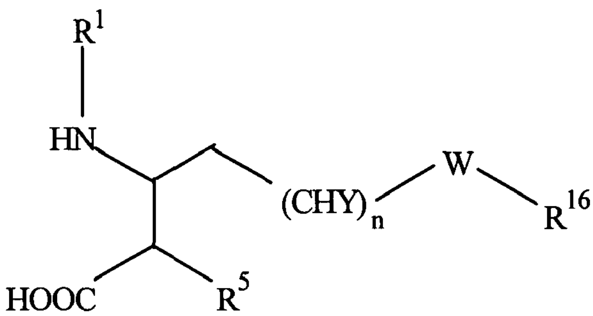

(XV)

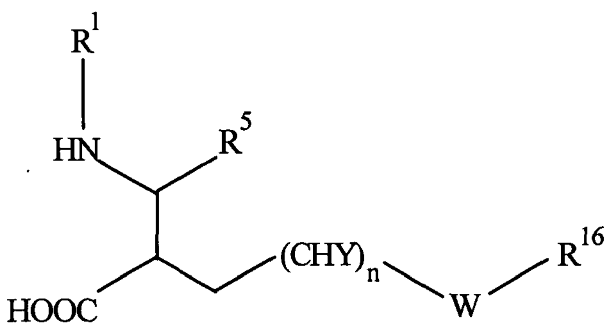

(XVI)

with

Hal-$(CHY)_{n'}$-X,

wherein $R^1$, $R^5$, X, Y, n and n' have the same meaning as indicated above in claim 1; W is O or S, $R^{16}$ is $C_1$ to $C_6$ alkyl and Hal is F, Cl, Br, or I.

**19.** Method for producing a binding compound comprising the step of

(i) selectively removing $R^3$ or $R^4$ from a orthogonally protected bifunctional amino acid and salts thereof having the formula (I), (II) or (III):

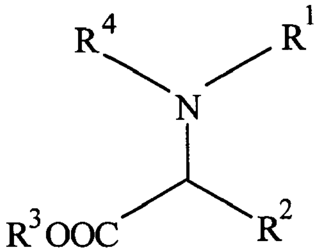

(I)

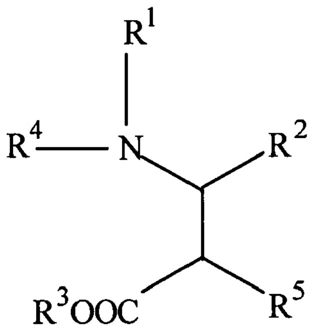

(II)

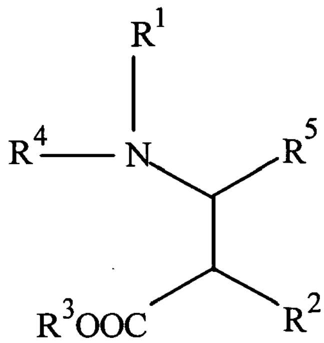

(III)

wherein,
$R^1$ and $R^2$ are independently of each other hydrogen, branched or linear $C_1$-$C_6$ alkyl, branched or linear substituted $C_1$-$C_6$ alkyl or -$CH_2$-$(CHY)_n$-W-$(CHY)_n$-X,;
W is CHY, S, O, $N(CH_3)$, $N(C_2H_5)$ or $N(C_3H_7)$;
X is COOH, $NH_2$, COZ, NHZ or Z;
Y is for each CHY independently hydrogen, methyl or halogen;
Z is an amino acid residue; a polypeptide; a protective group, which can be selectively removed in the presence of $R^3$ and $R^4$; a direct or indirect link to a metal chelating residue, a dye, a therapeutic compound or a surface; or a bond,
n is 0-6;
n' is 1-6, or n' is 0-6 under the proviso that W is CHY;
$R^3$ is a protective group, which can be selectively removed in the presence of $R^4$;
$R^4$ is a protective group, which can be selectively removed in the presence of $R^3$; and
$R^5$ is hydrogen, branched or linear $C_1$-$C_6$ alkyl, branched or linear substituted $C_1$-$C_6$ alkyl or an amino acid side chain residue.

**20.** Method for producing a binding compound according to claim 19, wherein $R_1$ is branched or linear $C_1$-$C_6$ alkyl or branched or linear substituted $C_1$-$C_6$ alkyl.

**21.** Method for producing a binding compound according to claims 19 or 20, wherein $R_2$ is branched or linear $C_1$-$C_6$ alkyl, branched or linear substituted $C_1$-$C_6$ alkyl or -$CH_2$-$(CHY)_n$-W-$(CHY)_n$-X.

**22.** Method for producing a binding compound according to any one of claims 19 to 22, wherein $R_1$ is branched or linear $C_1$-$C_6$ alkyl or branched or linear substituted $C_1$-$C_6$ alkyl and $R_2$ is -$CH_2$-$(CHY)_n$-W-$(CHY)_{n'}$-X.

**23.** Method for producing a binding compound according to any one of claims 19 to 23, wherein W is S, O or $N(CH_3)$.

**24.** Method for producing a binding compound according to any one of claims 1 to 5, wherein the amino acid residue is selected from the group of alanine-A, asparagine-A, cystine-A, asparagine-A, aspartic acid-A, glutamine-A, glutamic acid-A, phenylalanine-A, glycine-A, histidine-A, isoleucine-A, lysine-A, leucine-A, methionine-A, proline-A, arginine-A, serine-A, threonine-A, tryptophane-A, valine-A, tyrosine-A, tert-butyl glycine-A, N-methyl phenylalanine-A, lysine(GlyMeDOTA)-A Hcy-A, Hhc-A, Pen-A, Aib-A, Nal-A, Aca-A, Ain-A, Hly-A, Achxa-A, Amf-A, Aec-A, Apc-A, Aes-A, Aps-A, Abu-A, Nva-A, FD-A, WD-A, YD-A, Cpa-A, Thp-A, D-Nal-A, Dpg-A, Nle-A, (N-$CH_3$)Cys-A, (N-$CH_3$)Hcy-A, (N-$CH_3$)Tyr-A, (N-$CH_3$)Tty-A, (N-$CH_3$)Tyr-A($CH_2$ $CH_2$ SH), Thr(OH)-A, Ser(ol)-A, Asp(ol)-A, Glu(ol)-A, Gln(ol)-A, Asn(ol)-A, Phe(4-F)-A, Phe(4-$NH_2$)-A, ε-Lys-A, δ-Orn-A, γ-Dab-A, β-Dap-A, optionally comprising protected side chain residues,
wherein A is the amino or carboxyl group of the amino acid, a protected amino or carboxyl group or a direct or indirect link to a surface.

**25.** Method for producing a binding compound according to any one of claims 19 to 23, wherein the polypeptide is selected from the group consisting of a receptor ligand, an antibody, a single chain antibody or a binding fragment of an antibody or single chain antibody.

**26.** Method for producing a binding compound according to any one of claims 19 to 23, wherein the metal chelating residue is selected from the group consisting of

a) C$(pgp)^s$-(aa)-C$(pgp)^s$, wherein $(pgp)^s$ is hydrogen or a thiol protecting group and (aa) is any [alpha]- or [beta]-amino acid not comprising a thiol group;
b) a substance according to formula (IV) or (V)

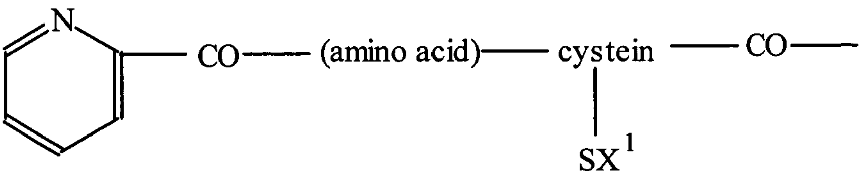

(IV)

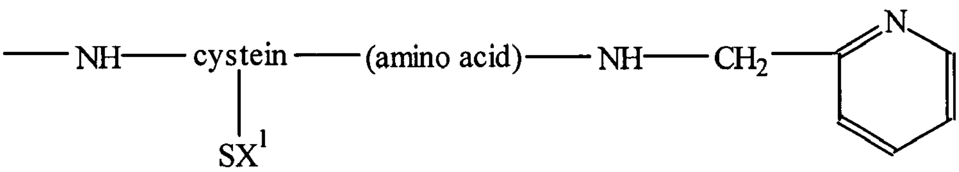

(V),

wherein $X^1$=H or a protecting group;
(amino acid)=any amino acid;
c) a substance according to formula (VI)

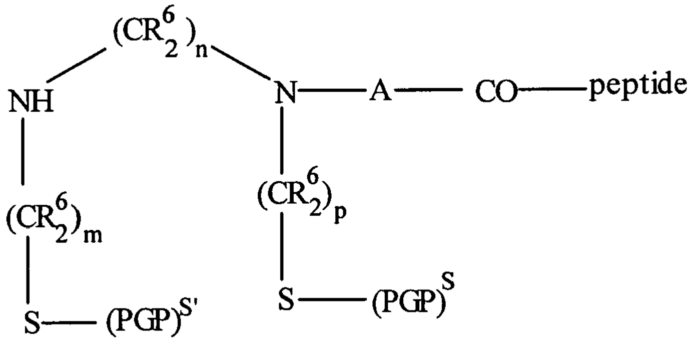

(VI),

wherein each $R^6$ is independently H, $CH_3$ or $C_2H_5$, each (pgp)' is independently a thiol protecting group or H; m, n and p are independently 2 or 3; A is linear $C_1$-$C_8$ alkyl, substituted linear $C_1$-$C_8$ alkyl, cyclic $C_3$-$C_8$ alkyl, substituted cyclic $C_3$-$C_8$ alkyl, aryl, substituted aryl, or a combination thereof; and

d) a substance according to formula (VII)

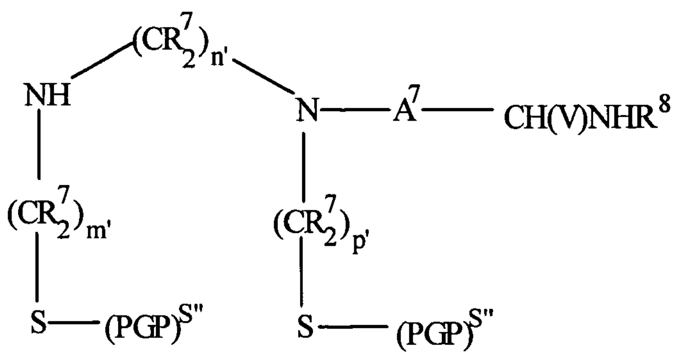

(VII),

wherein each $R^7$ is independently H, $CH_3$ or $C_2H_5$; each (pgp)S" is independently a thiol protecting group or H; m', n' and p' are independently 2 or 3; $A^1$ is linear $C_1$-$C_8$ alkyl, substituted linear $C_1$-$C_8$ alkyl, cyclic $C_3$-$C_8$ alkyl, substituted cyclic $C_3$-$C_8$ alkyl, aryl, substituted aryl, or a combination thereof; V is H or a CO link to X; $R^8$ is H or covalently linked to X;

e) diethylenetriaminepentaacetic acid (DTPA);

f) a derivative of DTPA having a formula (VIII)

$$(HOOCCH_2)_2N(CR^9_2)(CR^9_2)N(CH_2COOH)(CR^9_2)(CR^9_2)N(CH_2COOH)_2$$

(VIII),

wherein each $R^9$ is independently H, $C_1$ to $C_4$ alkyl, or aryl and one $R^9$ is covalently linked to X;

g) ethylenediaminetetraacetic acid (EDTA);

h) a derivative of EDTA having a formula (IX)

$$(HOOCCH_2)_2N(CR^{10}_2)(CR^{10}_2)N(CH_2COOH)_2$$

(IX),

wherein each $R^{10}$ is independently H, $C_1$ to $C_4$ alkyl, or aryl and one $R^{10}$ is covalently linked to X;
i) 1,4,7,10-tetraazacyclododecanetetraacetic acid and derivatives thereof;
j) a substance according to formula (X)

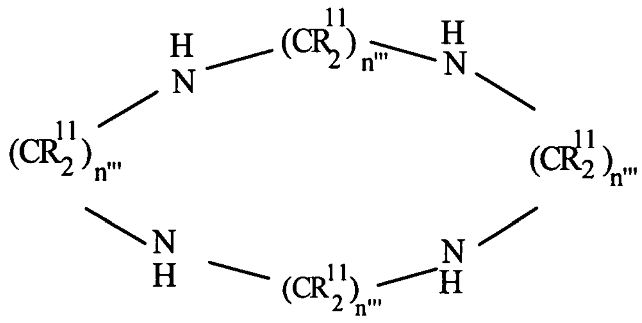

(X),

wherein n"' is an integer that is 2 or 3 and where each $R^{11}$ is independently H, $C_1$ to $C_4$ alkyl, or aryl and one $R^{11}$ is covalently linked to X;
m) a substance according to formula (XI) comprising a single thiol

$A^3$-$CZ^3(B^3)$-$\{C(R^{12}R^{13})\}_{n''}$-$X^3$ (XI),

wherein $A^3$ is H, HOOC-, $H_2$NOC-, -NHOC-, -OOC-, $R^{16}_2$NOC-, X-NHOC-, X-OOC-, or $R^{15}$; $B^3$ is H, SH, -$NHR^{14}$, -$N(R^{14})$-, X-$NR^{14}$- or $R^{15}$; $Z^3$ is H or $R^{15}$; $X^3$ is SH, -$NHR^{14}$, -$N(R^{14})$-, X-$NR^{14}$- or $R^{15}$; $R^{12}$, $R^{13}$, $R^{14}$ and $R^{15}$ are independently H, straight chain $C_1$-$C_8$ alkyl, e.g. methyl, ethyl, propyl, isopropyl, n-butyl, isobutyl, pentyl, hexyl, heptyl or octyl, branched chain $C_1$-$C_8$ alkyl, or cyclic $C_3$-$C_8$ alkyl, e.g. propyl, butyl, pentyl, hexyl, heptyl or octyl; n" is 0, 1 or 2; $R^{16}$ is $C_1$-$C_4$ alkyl, an amino acid, or a peptide comprising 2 to about 10 amino acids; and: (1) where $B^3$ is -$NHR^{14}$, X-$NR^{14}$- or -$N(R^{14})$-, $X^3$ is SH and n" is 1 or 2; (2) where $X^3$ is $NHR^{14}$, X-$NR^{14}$-, or -$N(R^{14})$-, $B^3$ is SH and n" is 1 or 2; (3) where $B^3$ is H or $R^{15}$, $A^3$ is HOOC-, $H_2$NOC-, X-NHOC-, X-OOC-, -NHOC-, or -OOC-, $X^3$ and n" is 0 or 1; (4) where $A^3$ is H or $R^{15}$, in cases where $B^3$ is SH, $X^3$ is - $NHR^{14}$, X-$NR^{14}$-, or -$N(R^{14})$- and where $X^3$ is SH, $B^3$ is $NHR^{14}$, X-$NR^{14}$- or - $N(R^{14})$ and n" is 1 or 2; (5) where $X^3$ is H or $R^{15}$, $A^3$ is HOOC-, $H_2$NOC-, - NHOC-, -OOC-, X-NHOC- or X-OOC- and $B^3$ is SH; (6) where $Z^3$ is methyl, $X^3$ is methyl, $A^3$ is HOOC-, $H_2$NOC-, -NHOC-, -OOC-, X-NHOC- or X-OOC- and $B^3$ is SH and n is 0; and (7) where $B^3$ is SH, $X^3$ is not SH and where $X^3$ is SH, $B^3$ is not SH, and
k) a substance according to formula (XII)

-βDap-Xaa-Cys-Zaa-A (XII),

wherein Xaa is an L-α-amino acid;
Zaa is an α-amino acid, an α-amino acid amide, an aminoethylether, a β-aminol, or a peptide containing from two to ten α-amino acids, said peptide having a carboxyl terminal α-amino acid, α-amino acid amide, aminoethylether, or β-aminol, and A is the amino or carboxyl group of the amino acid, a protected amino or carboxyl group or a direct or indirect link to a surface.

optionally comprising one or more protected side chain residues.

**27.** Method for producing a binding compound according to claim 26, wherein the metal chelating residue is selected from the group consisting of:

a) -βDap-Phe-Cys-Thr-Ser-A;
b) -βDap-Tyr-Cys-Thr(ol)-A;
c) -βDap-Phe(4-F)-Cys-Thr(ol)-A;
d) -βDap-Phe(4-$NH_2$)-Cys-Thr-Ser-A;
e) -βDap-Dab-Cys-Thr-A;
f) -βDap-Phe(4-NH2)-Cys-Thr-A;
g) -βDap-Phe(4-NH2)-Cys-Thr(ol)-A;
h) -βDap-His-Cys-Thr(ol)-A;
i) -βDap-Arg-Cys-Thr(ol)-A;
j) -βDap-Gly-Cys-Lys-$NH_2$-A;
k) -βDap-Ser-Cys-Thr(ol)-A;
l) -βDap-Dab-Cys-Thr(ol)-A;
m) -βDap-Gly-Cys-Thr(ol)-A;
n) -βDap-Dab-Cys-Ser(ol)-A;
o) -βDap-Ser-Cys-Thr-NH($CH_2CH_2O)_2$ $CH_2CH_2$NH-A;
p) -βDap-Orn-Cys-Thr(ol)-A;
q) -βDap-Dap-Cys-Thr(ol)-A;
r) -βDap-Lys-Cys-Thr(ol)-A; and
s) -βDap-Lys-Cys-NH-A;

optionally comprising one or more protected side chain residues.

**28.** Method for producing a binding compound according to claim 6 or 7, wherein the amino acid residue, the polypeptide or the metal chelating residue carries one or more protection group(s), which (is) are stable under conditions that remove $R^3$ and/or $R^4$.

**29.** Method for producing a binding compound according to any one of claims 1 to 9, wherein n is 1-3 and n' is 1-3.

**30.** Method for producing a binding compound according to any one of claims 19 to 29, wherein $R^3$ and $R^4$ are each selected $R^3$ and $R^4$ are each selected from a different group of protective groups selected from a protective group removable by a nucleophile, by acidic conditions, by hydrogenolysis, by mild base or by photolytic conditions.

**31.** Method for producing a binding compound according to any one of claim 30, wherein

(i) a protective group removed at acidic conditions, preferably at a pH between 4 and 6, which is selected from the group consisting of Boc or Trityl protecting groups;
(ii) a protective group removed by a nucleophile, which is selected from the group consisting of Fmoc or Dde protecting groups;
(iii) a protective group removed by hydrogenolysis consisting of the allyl type, the tert-butyl type, the benzyl type or Dmab (4,4-dimethyl-2,6-dicyclohexylidene)-3-methylbutyl]-amino}benzyl ester ;
(iv) a protective group removed by radiation, which is selected from the group consisting of nitroveratryloxy carbonyl, nitrobenzyloxy carbonyl, dimethyl dimethoxybenzyloxy carbonyl, 5-bromo-7-nitroindolinyl, o-hydroxy-α-methyl cinnamoyl, and 2-oxymethylene anthraquinone.

**32.** Method for producing a binding compound according to any one of claims 19 to 31, wherein $R^3$ is removed by hydrogenolysis, mild base or photolytic conditions and $R^4$ is removed by a nucleophile or acidic conditions.

**33.** Method for producing a binding compound according to any one of claims 19 to 31, wherein $R_3$ is selected from the group of protective groups consisting of a protective group of the allyl type, the tert-butyl type and the benzyl type and $R^4$ is selected from the group of protective groups consisting of Fmoc, Boc and Dde.

**34.** Method for producing a binding compound according to claim 19, wherein the amino acid has the formula IV:

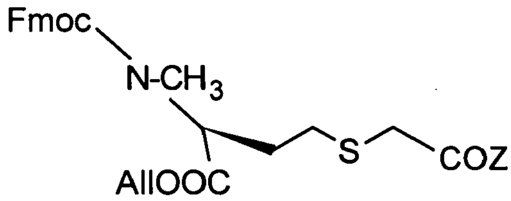

(IV)

**35.** Method for producing a binding compound according to any of claims 19 to 34, comprising the further step of:

(ii) coupling a monomeric building block to the deprotected carboxy or amino group of the amino acid, respectively.

**36.** Method for producing a binding compound according to claim 35, wherein the monomeric building block is selected from alanine-A, asparagine-A, cystine-A, asparagine-A, aspartic acid-A, glutamine-A, glutamic acid-A, phenylalanine-A, glycine-A, histidine-A, isoleucine-A, lysine-A, leucine-A, methionine-A, proline-A, arginine-A, serine-A, threonine-A, tryptophane-A, valine-A, tyrosine-A, tert-butyl glycine-A, N-methyl phenylalanine-A, lysine(GlyMeDOTA)-A Hcy-A, Hhc-A, Pen-A, Aib-A, Nal-A, Aca-A, Ain-A, Hly-A, Achxa-A, Amf-A, Aec-A, Apc-A, Aes-A, Aps-A, Abu-A, Nva-A, FD-A, WD-A, YD-A, Cpa-A, Thp-A, D-Nal-A, Dpg-A, Nle-A, ($N-CH_3$)Cys-A, ($N-CH_3$)Hcy-A, ($N-CH_3$)Tyr-A, ($N-CH_3$)Tty-A, ($N-CH_3$)Tyr-A($CH_2$ $CH_2$ SH), Thr(OH)-A, Ser(ol)-A, Asp(ol)-A, Glu(ol)-A, Gln(ol)-A, Asn(ol)-A, Phe (4-F)-A, Phe(4-$NH_2$)-A, ε-Lys-A, δ-Orn-A, γ-Dab-A, β-Dap-A, a polypeptide and a ligand.

**37.** Method for producing a binding compound according to claim 36, wherein the ligand is selected from the group consisting of an antibody, a single chain antibody, a binding fragment of an antibody or single chain antibody and a peptide ligand.

**38.** Method for producing a binding compound according to any one of claims 35 to 37, wherein the monomeric building block comprises a protective group(s) $R^3$ and/or $R^4$ and optionally one or more protective group(s) which is (are) stable under conditions that remove $R^3$ and/or $R^4$.

**39.** Method for producing a binding compound according to claim 35 to 38, comprising the further steps of:

(iii) selectively removing the protective group $R^3$ or $R^4$ from the monomeric building block or the amino acid, and
(iv) coupling a further monomeric building block, optionally comprising (a) protective group(s) $R^3$ and/or $R^4$ to the deprotected monomeric building block or amino acid.

**40.** Method for producing a binding compound according to claim 39, wherein the steps (iii) and (iv) are repeated one or more times, optionally after the last coupling step (iv) step (iii) is carried out once and/or a cyclisation reaction is carried out.

**41.** Method for producing a binding compound according to any one of claims 35 to 40, wherein two monomeric building blocks, optionally comprising (a) protective group(s) $R^3$ and/or $R^4$, are added subsequently or simultaneously to both the deprotected carboxy and to the deprotected amino group of the amino acid.

**42.** Method for producing a binding compound according to claim 41, comprising the further steps of:

(v) selectively removing the protective group $R^3$ and/or $R^4$ from one of the monomeric building blocks, and
(vi) coupling a further monomeric building block, optionally comprising (a) protective group(s) $R^3$ and/or $R^4$ to the deprotected monomeric building block.

**43.** Method for producing a binding compound according to claim 42, wherein the steps (v) and (vi) are repeated one or more times, optionally after the last coupling step (vi) step (v) is carried out once and/or a cyclisation reaction is carried out.

**44.** Method for producing a binding compound according to any one of claims 19 to 43, comprising the following steps: removing $R^4$, coupling Phe-$R^4$, removing $R^3$, coupling Tyr-$R^3$, removing $R^4$, coupling Thr-$R^4$, removing $R^4$, coupling Lys-$R^4$, removing $R^4$, coupling Trp-$R^4$, removing $R^3$ and $R^4$, cyclisation and optionally cleavage from a surface and/or removing one or more protective group(s), which is (are) stable under conditions that remove $R^3$ and/or $R^4$.

**45.** Method for producing a binding compound according to any one of claims 19 to 44, wherein one or more monomeric building blocks are coupled to produce a cyclic peptide with the sequence according to formula (XIII):

cyclo[$X^3$-DTrp-Lys-$X^4$-$X^5$-$X^6$] (XIII),

wherein
$X^3$ is diphenyl-Ala, (1)Nal, (2)Nal, (4)Pal, Phe(4-F), Thioproline, Trp or Tyr;
$X^4$ is βAla(cyclopropyl), diaminopropanoic acid, Thr or Val;
$X^5$ is an amino containing a side-chain as either the D or L isomer, capable of conjugating to a metal chelating residue, a dye, or a therapeutic compound, or a natural or unnatural α-amino acid, or a N- alkyl α-amino acid;
$X^6$ is a radical of an amino acid according to formula (I), (II) or (III).

**46.** Method for producing a binding compound according to claim 45, wherein one or more monomeric building blocks are coupled to produce a cyclic peptide with the sequence:

a) cyclo[Tyr-DTrp-Lys-Thr-Phe-(NMe)hCys];
b) cyclo[1Nal-DTrp-Lys-Thr-Met-(NMe)Phe];
c) cyclo[Trp-DTrp-Lys-Thr-Met-(NMe)Phe];
d) cyclo[1Nal-DTrp-Lys-Val-Met-(NMe)Phe];
e) cyclo[Phe(4-F)-DTrp-Lys-Thr-Met-(NMe)Phe];
f) cyclo[Tyr-DTrp-Lys-Val-Met-(NMe)Phe];
g) cyclo[1Nal-DTrp-Lys-Thr-Lys(GlyMeDOTA)-(NMe)Phe];
h) cyclo[Tyr-DTrp-Lys-Thr-Met-(NMe)Phe];
i) cyclo[2Nal-DTrp-Lys-Thr-Met-(NMe)Phe];
j) cyclo[Tyr-DTrp-Lys-Thr-Met-Tpi];
k) cyclo[Tyr-Dtrp-Lys-BAla(cyclopropyl)-Met-(NMe)Phe];
l) cyclo[Tyr-DTrp-Lys-Dpr-Met-(NMe)Phe];
m) cyclo[ThioPro-DTrp-Lys-Thr-Met-Phe];
n) cyclo[DiphenylAla-DTrp-Lys-Thr-Met-(NMe)Phe];
o) cyclo[(4)Pal-DTrp-Lys-Thr-Met-(NMe)Phe].

**47.** Method for producing a binding compound according to any one of claims 19 to 46, further comprising the steps of:

(vii) optionally purifying the binding compound and
(viii) radiolabeling the binding compound with $^{186}$Re, $^{188}$Re, $^{212}$Bi, $^{213}$Bi, $^{90}$Y, $^{153}$Sm, $^{47}$Sc, $^{68}$Ga, $^{94m}$Tc, $^{99m}$Tc , $^{67}$Cu, $^{166}$Ho, $^{223}$Ra , $^{225}$Ac, $^{18}$F, $^{125}$I, $^{131}$I, $^{123}$I, or $^{211}$At or a salt thereof.

**48.** Method for producing a binding compound according to any one of claims 19 to 47, further comprising the steps:

(ix) optionally purifying the binding compound and
(x) admixing the binding compound with a pharmaceutically acceptable carrier, additive(s), and/or buffer.

**49.** Use of a binding compound producible according to any one of claims 19 to 48, for the production of a therapeutic for the treatment of proliferative diseases, infectious diseases, vascular diseases, rheumatoid diseases, inflammatory diseases, immune diseases, in particular autoimmune diseases and allergies.

**50.** Use of a binding compound producible according to any one of claims 19 to 48, for the production of a diagnostic for the diagnosis of proliferative diseases, infectious diseases, vascular diseases, rheumatoid diseases, inflammatory diseases, immune diseases, in particular autoimmune diseases and allergies.

**Fig. 1**

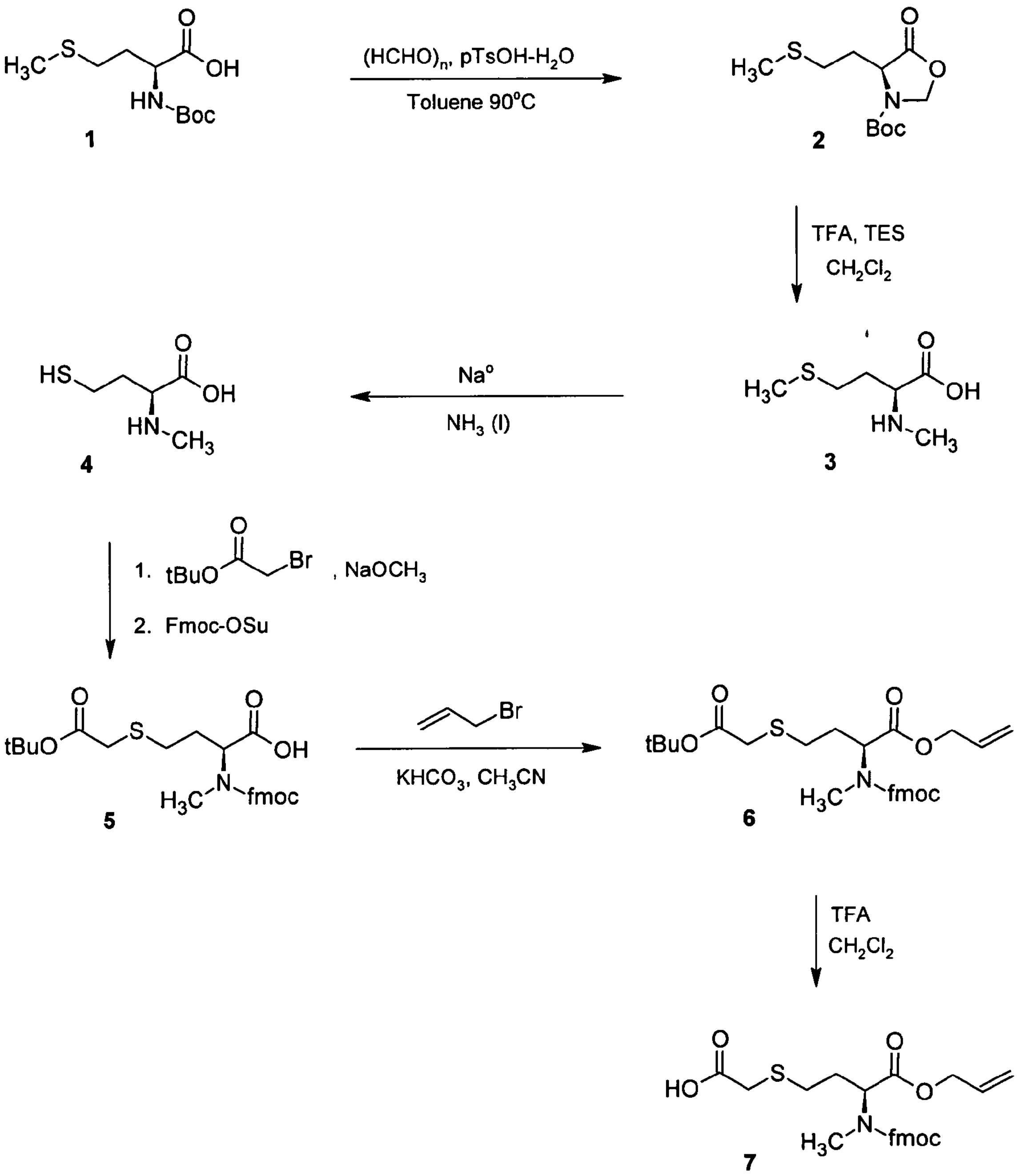
1
$(HCHO)_n$, pTsOH-$H_2O$
Toluene 90°C
2
TFA, TES
$CH_2Cl_2$
3
Na°
$NH_3$ (l)
4
1.
, $NaOCH_3$
2. Fmoc-OSu
5
$KHCO_3$, $CH_3CN$
6
TFA
$CH_2Cl_2$
7

European Patent Office

# PARTIAL EUROPEAN SEARCH REPORT

which under Rule 45 of the European Patent Convention shall be considered, for the purposes of subsequent proceedings, as the European search report

Application Number
EP 05 00 9363

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DING, VERA, LIANG, ZHAO, LEONARD, JOULLIÉ: "Structure-activity relationships of side-chain modified didemnins" BIOORGANIC & MEDICINAL CHEMISTRY LETTERS, vol. 11, 2001, pages 231-234, XP002371032 * compound 9 * ----- | 1-16, 18-48 | INV. C07C217/22 C07C269/04 C07C269/06 C07K7/06 C07K7/52 |
| X | KAWABATA, KAWAKAMI, MAJUMDAR: "Asymmetric cyclization via memory of chirality: A concise access to cyclic amino acids with a quaternary sterreocenter" JOURNAL OF THE AMERICAN CHEMICAL SOCIETY, vol. 125, 2003, pages 13012-13013, XP002371033 * compounds 7, 9 * ----- -/-- | 1-16, 18-48 | |

TECHNICAL FIELDS SEARCHED (IPC)

C07C
A61K
C07K

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 19 July 2006 | Pérez Carlon, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

8

European Patent Office

# PARTIAL EUROPEAN SEARCH REPORT

Application Number
EP 05 00 9363

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| X | BELOV, RANDS, RADDATZ KRÜGER, NIKOLSKAYA, SOKOLOV, DE MEIJERE: "First enantioselective synthesis of the novel antiinfective TAN-1057A via its aminomethyl-substituted dihydropyrimidinone heterocycle" TETRAHEDRON, vol. 60, 2004, pages 7579-7589, XP002371034 * compound 10 * ----- | 1-16, 18-48 | |
| X | BAVETSIAS, JACKMAN, MARRIOTT, KIMBELL, GIBSON, BOYLE, BISSET: "Folate-based inhibitors of thymidylate synthase: Synthesis and antitumor activity of gamma-linked sterically hindered dipeptide analogues of 2-desamino-2-methyl-N10-propargyl-5,8-dideazafolic acid (ICI 198583)" JOURNAL OF MEDICINAL CHEMISTRY, vol. 40, no. 10, 1997, pages 1495-1510, XP002371035 * compounds 4A-4C, 40, 46 * ----- | 1-16, 18-48 | TECHNICAL FIELDS SEARCHED (IPC) |
| X | ROBILLARD, VAN ALPHEN, MEEUWENOORD, JANSEN, VAN DER MAREL, VAN BOOM, REEDIJK: "Solid-phase synthesis of peptide-platinum complexes using platinum-chelatin boulding blocks derived from amino acids." NEW JOURNAL OF CHEMISTRY, vol. 29, January 2005 (2005-01), pages 220-225, XP002371036 * compound 8A * ----- | 1-16, 18-48 | |
| | -/-- | | |

EPO FORM 1503 03.82 (P04C10)

8

European Patent Office

# PARTIAL EUROPEAN SEARCH REPORT

Application Number
EP 05 00 9363

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YANG, CHIU: "Solid phase synthesis of Fmoc N-methyl amino acids: application of the Fukuyama amine synthesis" TETRAHEDRON LETTERS, vol. 38, 1997, pages 7303-7310, XP002371037 P. 7308, first line, second and third compounds ----- | 1-16, 18-48 | |
| X | GALPIN, MOHAMMED, PATEL, PRIESTLEY: "Synthesis of two linear octapeptide fragments of cyclosporin by stepwise and fragment condensation strategies" TETRAHEDRON, vol. 44, 1988, pages 1763-1772, XP002371038 Z-(Me)Val-OBut, Z-(Me)Ley-OBut, p. 1767, l. 41 ----- | 1-16, 18-48 | TECHNICAL FIELDS SEARCHED (IPC) |
| X | HUMPRHEY, AGGEN, CHAMBERLIN: "Total synthesis of the serine-threonine phosphatase inhibitor microcystin-LA" JOURNAL OF THE AMERICAN CHEMICAL SOCIETY, vol. 118, 1996, pages 11759-11770, XP002371039 * compounds 16, 17 * ----- | 1-16, 18-48 | |
| X | ZOU, WEI, CAI, MA: "Synthesis of an oxazoline analogue of Apratoxin A" ORGANIC LETTERS, vol. 5, no. 19, 2003, pages 3503-3506, XP002371040 * compound 17 * ----- | 1-16, 18-48 | |

-/--

8

EPO FORM 1503 03.82 (P04C10)

European Patent Office

# PARTIAL EUROPEAN SEARCH REPORT

Application Number
EP 05 00 9363

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HLAVACEK, MARCOVA, BUDESINSKY, SLANINOVA: "1-deamino-1(15)-carba and dicarba analogues of endothelin-1" COLLECT. CZECH. CHEM. COMMUN., vol. 65, 2000, pages 407-424, XP009063034 * compound 5 * | 1-50 | |
| A | LIN H ET AL: "Macrolactamization of Glycosylated Peptide Thioesters by the Thioesterase Domain of Tyrocidine Synthetase" December 2004 (2004-12), CHEMISTRY AND BIOLOGY, CURRENT BIOLOGY, LONDON, GB, PAGE(S) 1635-1642 , XP004689687 ISSN: 1074-5521 * figure 1 * | 17 | TECHNICAL FIELDS SEARCHED (IPC) |
| X | KELLEMAN, MATTERN, PIERSCHBACHER, GOODMAN: "Incorporation of thioether building blocks into an alpha.v.beta.3-specific RGD peptide: Synthesis and biological activity" BIOPOLYMERS (PEPTIDE SCIENCE), vol. 71, 2003, pages 686-695, XP002390990 * compound 12 * | 1-48 | |
| X | KIM, CARROLL, LEE: "Preparation and synthetic applications of sterically hindered secondary amines" SYNTHETIC COMMUNICATIONS, vol. 27, no. 14, 1997, pages 2505-2515, XP009069500 Scheme 4, second compound. | 1-16, 18-33, 35-38 | |

-/--

8

EPO FORM 1503 03.82 (P04C10)

European Patent Office

# PARTIAL EUROPEAN SEARCH REPORT

Application Number
EP 05 00 9363

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| X | ROSTOVTSEVA, KIRYUSHIKIN KHOKHLOV: "L-beta-lysine peptides IV. Structure and synthesis of dipeptide isolated from Streptotrhricin C" J. GEN. CHEM. USSR, vol. 41, 1971, pages 1618-1622, XP009069499 Scheme 2, middle compound. ----- | 1-16, 18-33, 35-48 | |
| X | SIBI, PATIL: "Enantioselective H-atom transfer reactions: A new methodology for the synthesis of beta2-amino acids" ANGEW. CHEM., vol. 116, 2004, pages 1255-1258, XP002390991 Scheme 2, reaction product with FmocCl, Py. ----- | 1-16, 18-33, 35-48 | TECHNICAL FIELDS SEARCHED (IPC) |
| X | BALDWIN, ADLINGTON, O'NEIL, SCHOFIELD, SPIVEY, SWEENEY: "The ring opening of aziridine-2-carboxylate esters with organometallic reagents" J. CHEM. SOC. CHEM. COMMUN, 1989, pages 1852-1854, XP009069530 * compound 5 * ----- | 1-16, 18-33, 35-48 | |
| X | PU, MA: "Asymmetric total synthesis of (-)-alkaloid 223A and its 6-epimer" J. ORG. CHEM., vol. 68, 2003, pages 4400-4405, XP002390992 * compounds 5, 6 * ----- | 1-16, 18-33, 35-48 | |
| | -/-- | | |

EPO FORM 1503 03.82 (P04C10)

8

European Patent Office

# PARTIAL EUROPEAN SEARCH REPORT

Application Number
EP 05 00 9363

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BISCHOFF, DAVID, MARTIN, MEUDAL, ROQUES, FOURNIÉ-ZALUSKI: "2,4-Dinitrophenyl 4-methoxybenzyl disulfide: A new efficient reagent for the electrophilic sulfenylation of b-amino ester enolates" J. ORG. CHEM., vol. 62, 1997, pages 4848-4850, XP002390993 * compounds 3A, C, E, F, G * ----- | 1-16, 18-33, 35-48 | |
| X | MARINI, ROUMESTANT, VIALLEFONT, RAZAFINDRAMBOA, BONATO, FOLLET: "Synthesis of enantiomerically pure b- and g-amino acids from aspartic and glutamic acid derivatives" SYNTHESIS, 1992, pages 1104-1108, XP002390994 * compounds 14A, 14D * ----- | 1-16, 18-33, 35-48 | TECHNICAL FIELDS SEARCHED (IPC) |
| X | BRADS, ENDERMANN, GAHLMANN, KRÜGER, RADDATZ, SOLEFUSS, BELOV, NIZAMOV, SOKOLOV, DE MEIJERE: "Novel antibiotics for the treatment of gram-positive bacterial infections" J. MED. CHEM., vol. 45, 2002, pages 4246-4253, XP002390995 * compounds 10, 10-ME, 11 * ----- | 1-16, 18-33, 35-48 | |
| X | HABERMEHL, WILHELM: "Synthese von N-[(3-Ethoxycarbonylmethyl)-cyclohexyl]-azetidin-2-yl-propansäurehydrochlorid" Z. NATURFORSCH. B, vol. 47, 1992, pages 1779-1784, XP009069502 * compound 9 * ----- | 1-16, 18-33, 35-48 | |

-/--

European Patent Office

# PARTIAL EUROPEAN SEARCH REPORT

Application Number
EP 05 00 9363

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DAVID, BISCHOFF, MEUDAL, MOTHÉ, DE MOTA, DANASCIMENTO LLORENS-CORTES, FOURNIÉ-ZALUSKI, ROQUES: "Investigation of subsite preferences in Aminopeptidase A (EC 3.4.11.7) led to the desing of the first highly potent and selective inhibitors of this enzyme" J. MED. CHEM., vol. 42, 1999, pages 5197-5211, XP002390996 * compounds 4A, 4B, 10 * ----- | 1-16, 18-33, 35-48 | |
| X | US 6 358 491 B1 (LISTER-JAMES JOHN ET AL) 19 March 2002 (2002-03-19) * claims 20,21,23,37,38 * ----- | 49,50 | |

TECHNICAL FIELDS SEARCHED (IPC)

8

EPO FORM 1503 03.82 (P04C10)

European Patent Office

## INCOMPLETE SEARCH SHEET C

Application Number
EP 05 00 9363

Claim(s) searched completely:
15, 17

Claim(s) searched incompletely:
1-14, 16, 18-50

Reason for the limitation of the search:

The application is considered as lacking support in the sense of Arts. 83 and 84 EPC. The only example provided in the description refers to the obtention of the compound subject-matter of claim 17. Extrapolation to the general formulas of claim 1 or to the synthesis of peptides, most of them not even containing the structural unity of the compound of the example contravenes the requirements of Arts. 83 and 84 EPC.

Initial stages of search have also revealed a very large number of documents relevant for the novelty of claim 1.

European Patent Office

Application Number

EP 05 00 9363

## CLAIMS INCURRING FEES

The present European patent application comprised at the time of filing more than ten claims.

- [ ] Only part of the claims have been paid within the prescribed time limit. The present European search report has been drawn up for the first ten claims and for those claims for which claims fees have been paid, namely claim(s):

- [ ] No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

- [X] All further search fees have been paid within the fixed time limit. The present European search report has been drawn up for all claims.

- [ ] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [ ] None of the further search fees have been paid within the fixed time limit. The present European search report has been drawn up for those parts of the European patent application which relate to the invention first mentioned in the claims, namely claims:

European Patent Office

# LACK OF UNITY OF INVENTION SHEET B

Application Number
EP 05 00 9363

The Search Division considers that the present European patent application does not comply with the requirements of unity of invention and relates to several inventions or groups of inventions, namely:

1. claims: 1(part)-16(part), 17, 18(part)-33(part), 34, 35(part)-48(part)

   Orthogonally protected alpha amino-acids of the general formula (I), their synthesis and uses.

   ---

2. claims: 1(part)-16(part), 18(part)-33(part), 35(part)-38(part)

   Orthogonally protected beta-aminoacids of the genreal formula (II), their synthesis and uses.

   ---

3. claims: 1(part)-16(part), 18(part)-33(part), 35(part)-38(part)

   Orthogonally protected beta-aminoacids of the general formula (III), their synthesis and uses.

   ---

4. claims: 49, 50

   Use of known compounds obtainable according to claims 19-48.

   ---

# ANNEX TO THE EUROPEAN SEARCH REPORT ON EUROPEAN PATENT APPLICATION NO. EP 05 00 9363

This annex lists the patent family members relating to the patent documents cited in the above-mentioned European search report.
The members are as contained in the European Patent Office EDP file on
The European Patent Office is in no way liable for these particulars which are merely given for the purpose of information.

19-07-2006

| Patent document cited in search report | Publication date | Patent family member(s) | Publication date |
|---|---|---|---|
| US 6358491 B1 | 19-03-2002 | AU 782873 B2 | 08-09-2005 |
| | | AU 4865901 A | 25-06-2001 |
| | | CA 2383936 A1 | 21-06-2001 |
| | | EP 1208116 A2 | 29-05-2002 |
| | | WO 0144177 A2 | 21-06-2001 |
| | | JP 2003517037 T | 20-05-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4310518 A
- US 4486415 A
- EP 0143307 A
- EP 0222578 A
- US 5708135 A
- US 5770687 A
- US 5654272 A
- US 5681541 A
- US 5788960 A
- US 5811394 A
- US 5720934 A
- US 5776428 A
- US 5780007 A
- US 5922303 A
- US 6093383 A
- US 6086849 A
- US 5965107 A
- US 5300278 A
- US 5350837 A
- US 5589576 A
- US 5679778 A
- US 5879659 A
- WO 0061194 A
- WO 0071162 A
- WO 0152746 A
- WO 0152743 A
- WO 0162156 A
- US 5443815 A
- US 5807537 A
- US 5814297 A
- US 5866097 A
- US 5997844 A
- US 6074627 A
- WO 9531221 A
- WO 9533497 A


### Non-patent literature cited in the description


- **ALESSI P et al.** *Biochim. Biophys. Acta,* 2004, vol. 1654, 39-49
- **NANDA A ; ST. CROIX B.** *Curr. Opin. Oncol.,* 2004, vol. 16, 44-49
- **ALBERICIO F.** Peptide Science. John Wiley & Sons, Inc, 03 November 2000, vol. 55, 123-139